(19) Europäisches Patentamt
European Patent Office
Office européen des brevets

(11) Publication number : **0 521 692 A2**

(12) # EUROPEAN PATENT APPLICATION

(21) Application number : **92306020.6**

(22) Date of filing : **30.06.92**

(51) Int. Cl.[5] : **A61K 31/70,** A61K 47/48, A61K 39/00, // C12M3/00

(30) Priority : **02.07.91 US 724983**
**12.11.91 US 789969**
**19.02.92 US 836978**

(43) Date of publication of application :
**07.01.93 Bulletin 93/01**

(84) Designated Contracting States :
**AT BE CH DE DK ES FR GB GR IT LI LU MC NL PT SE**

(71) Applicant : **The Biomembrane Institute**
**201 Elliott Avenue West Suite 305**
**Seattle Washington 98119 (US)**

(72) Inventor : **Hakomori, Sen-itiroh, c/o The Biomembrane Inst.**
**201 Elliott Avenue, Suite 305**
**Seattle, Washington 98119 (US)**
Inventor : **Handa, Kazuko, c/o The Biomembrane Institute**
**201 Elliott Avenue, Suite 305**
**Seattle, Washington 98119 (US)**
Inventor : **Toyokuni, Tatsushi, c/o The Biomembrane Institute**
**201 Elliott Avenue, Suite 305**
**Seattle, Washington 98119 (US)**
Inventor : **Nudelman, Edward, c/o The Biomembrane Institute**
**201 Elliott Avenue, Suite 305**
**Seattle, Washington 98119 (US)**

(74) Representative : **Rickard, Timothy Mark Adrian et al**
**Brookes & Martin, High Holborn House, 52/54 High Holborn**
**London WC1V 6SE (GB)**

(54) **Inhibition of tumor cell metastasis potential and invasiveness by chemically-defined oligosaccharides, their derivatives, mimetics and antibodies directed to them.**

(57) Methods and compositions for inhibiting metastasis potential of tumor cells are provided. Metastasis potential of tumor cells may be inhibited by an agent selected from the group consisting of mimetics of monosialosyl-$Le^a$ I, monosialosyl-$Le^a$ II, disialosyl-$Le^a$, and sialosyl $Le^x$, the agent inhibiting the metastasis potential of the preparation. The present invention also discloses conjugates comprising the agent and poly(ethylene glycol). Methods for inhibiting GMP-140-mediated or ELAM-1-mediated cell aggregation or adhesion are also provided. The aggregation or adhesion at a tumor site can be inhibited by an agent selected from the group consisting of mimetics of monosialosyl-$Le^a$ I, monosialosyl-$Le^a$ II, disialosyl-$Le^a$, and sialosyl $Le^x$, the agent inhibiting the cell aggregation or adhesion.

EP 0 521 692 A2

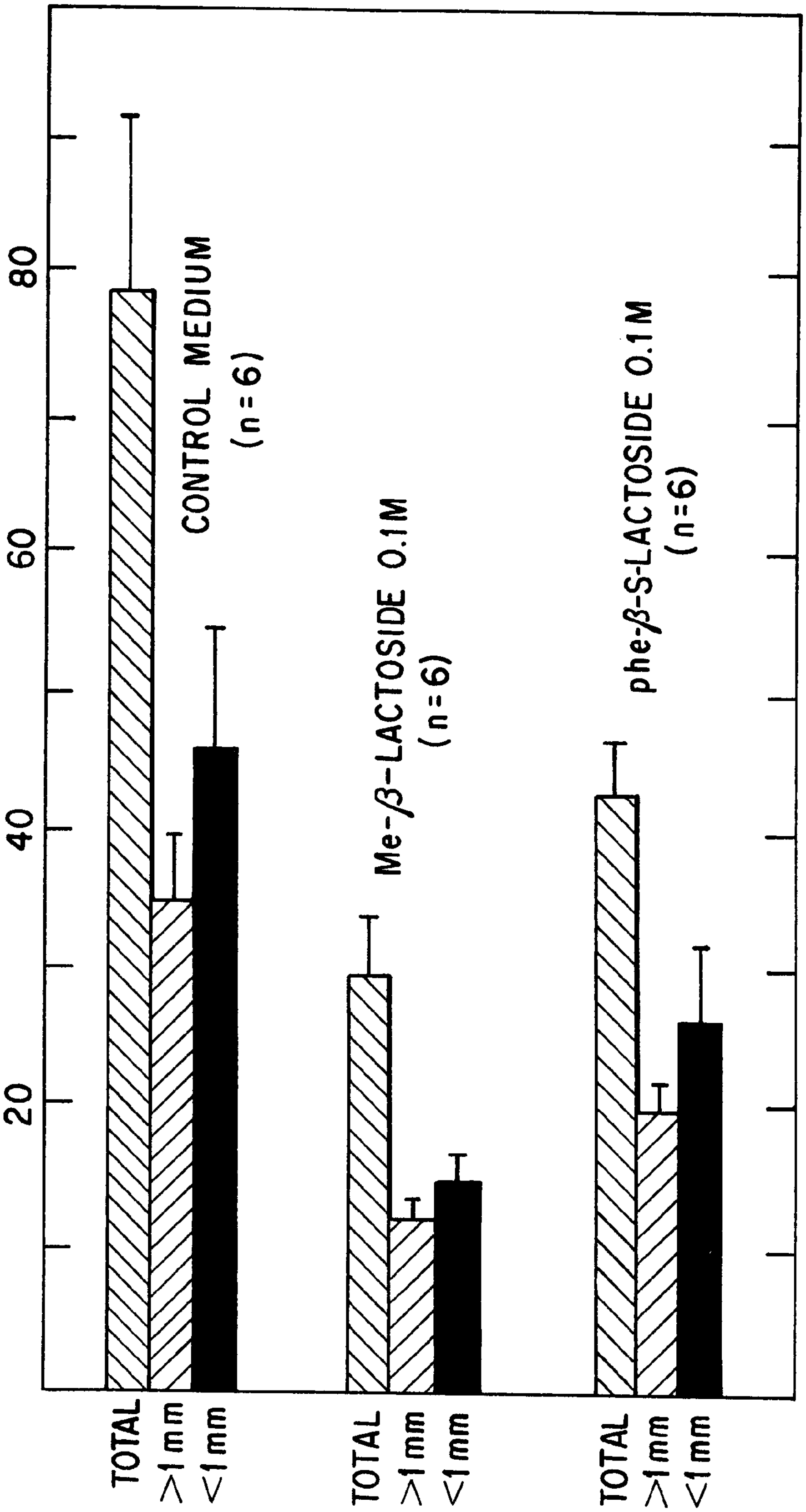
CONTROL MEDIUM
(n=6)
Me-β-LACTOSIDE 0.1 M
(n=6)
phe-β-S-LACTOSIDE 0.1 M
(n=6)
TOTAL
>1 mm
<1 mm
TOTAL
>1 mm
<1 mm
TOTAL
>1 mm
<1 mm
20
40
60
80
20
40
60
80

FIG. 1

## Cross-Reference To Related Applications

This application is a continuation-in-part of pending U.S. Application Serial No. 07/789,969, filed November 12, 1991 which is a continuation-in-part of pending U.S. Application Serial No. 07/724,983, filed July 2, 1991, which is a continuation-in-part of U.S. application Serial No. 07/575,539, filed August 30, 1990 (abandoned). All three applications are expressly incorporated herein by reference.

## Technical Field

The present invention is generally directed toward the inhibition of tumor cell metastases and invasiveness, and more specifically, toward such inhibition through the use of tumor-associated carbohydrate antigens, their oligosaccharide derivatives, mimetics of the tumor-associated carbohydrate antigens, and antibodies directed to the tumor-associated carbohydrate antigens.

## Background of the Invention

Despite enormous investments of financial and human resources, cancer remains one of the major causes of death. Current cancer therapies cure only about fifty percent of the patients who develop a malignant tumor. In most human malignancies, metastasis is the major cause of death.

Metastasis is the formation of a secondary tumor colony at a distant site. It is a multistep process of which tumor invasion is the first step. Tumor cells locally invade host tissue barriers, such as the epithelial basement membrane, to reach the interstitial stroma, where they gain access to blood vessels (or lymphatic channels) for further dissemination. After invading the endothelial layer of the vessel wall, the circulating tumor cells are dislodged into the circulation and arrest in the precapillary venules of the target organ by adherence to endothelial cell lumenal surfaces, or exposed basement membranes. The tumor cells again invade the vascular wall to enter the organ parenchyma. Finally, the extravasated tumor cell grows in a tissue different from where it originated.

In most human malignancies, distant metastases are often too small to be detected at the time the primary tumor is treated. Furthermore, widespread initiation of metastatic colonies usually occurs before clinical symptoms of metastatic disease are evident. The size and age variation in metastases, their dispersed anatomical location, and their heterogeneous composition are all factors that hinder surgical removal and limit the concentration of anticancer drugs that can be delivered to the metastatic colonies.

Due to the difficulties in the current approaches to the treatment and prevention of metastases, there is a need in the art for improved methods and compositions for inhibiting metastasis potential of tumor cells. The present invention fills this need, and further provides other related advantages.

## Summary of the Invention

Briefly stated, the present invention provides a biochemical method of inhibiting metastatic potential and invasiveness of tumor cells based on blocking tumor cell adhesion by carbohydrate structures or antibodies directed to them. The rationale for this approach is as follows:

i) In model experiments with mouse melanoma B16 variants with high and low metastatic potential, high metastatic variants BL6 and F10 express more GM3 than low- or non-metastatic F1 or WA4. Adhesion of high metastatic variants on endothelial cells is greater than low metastatic variants and the adhesion is inhibited by Me-β-lactoside, GM3 or LacCer (in their liposomes) or other lactoside derivations. These sugars and derivatives also inhibit B16 melanoma metastatic potential.

ii) In human cancers, patients whose primary tumors express defined tumor-associated carbohydrate antigens, such as H/$Le^y$/$Le^b$ (defined by monoclonal antibody MIA-15-5), sialosyl Tn (defined by monoclonal antibody TKH2, sialosyl-$Le^x$ (defined by monoclonal antibody FH6, or SNH 3 or 4), showed a much shorter survival rate than those of patients whose primary tumors do not express or which weakly express those antigens.

iii) Those tumor-associated carbohydrate antigens (GM3 in mouse melanoma model) and H/$Le^b$/$Le^y$, sialosyl-$Le^x$, sialosyl-Tn in human tumors are essentially adhesion molecules which are recognized by target cells, particularly platelet or endothelial cells.

iv) Interaction of tumor cells with endothelial cells and platelets is mediated by LECCAM (or selectin), ELAM-1 or GMP-140, which are expressed on activated endothelial cells and activated platelets. Sialosyl-$Le^x$ antigen has been known to be recognized by these LECCAMS.

v) GMP-140, whose expression on platelet or endothelial cells is induced by thrombin, ADP or (AMP) phorbol ester, may play an important role in platelet-tumor cell interaction and mediate tumor cell metastases. While the epitope recognized by this selectin was previously identified as sialosyl-$Le^x$ (Polley et al, Proc. Natl. Acad. Sci. 88:6224, 1991), the present inventors have now identified that sialosyl- $Le^a$ (also known as monosialosyl-$Le^a$ I), monosialosyl-$Le^a$ II (a positional isomer of sialosyl-$Le^a$), and disialosyl-$Le^a$ are also recognized by GMP-140. GMP-140 binds to sialosyl $Le^a$ better than to sialosyl-$Le^x$. Handa, et al., Biochem Biophys Res Comm, 181:1223, 1991 have also identified sialosyl-$Le^a$ as an epitope recognized by GMP-140.

vi) ELAM-1, whose expression on endothelial cells is induced by interleukin-1, TGF-β, TNF-α, or lipopolysaccharide, may play an important role in endothelial cell-tumor cell interaction and mediate tumor cell metastatis. While the epitopes recognized by this selection were previously identified as sialosyl-$Le^x$ and sialosyl-$Le^a$ (Phillips et al., Science 250:1130, 1990; Berg et al., J Biol Chem 266:14869, 1991; Takada et al., Biochem Biophys Res Commun 179:713, 1991), the present inventors have now identified that monosialosyl-$Le^a$ II and disialosyl-$Le^a$ are also recognized by ELAM-1 and other selectives, particularly in a dynamic flow system.

vii) Human colon tumor cells showing differential expression of metastatic potential in nude mice showed a close correlation with the expression of sialosyl-$Le^x$ , i.e., cells with high metastatic potential expressed high sialosyl-$Le^x$ and vice versa.

viii) Human endothelial cells are characterized by high expression of H (Fucα1→2Gal) and many types of human cancers are characterized by expression of $Le^y$, H, or $Le^b$ defined by monoclonal antibody MIA-15-5. Interaction of H to $Le^y$ or H to H has been clearly established, therefore, those human tumors expressing H/$Le^y$/$Le^b$ may adhere on H-expressing endothelial cells which are mediated by $Le^y$-H or H-H interaction.

ix) Monoclonal antibodies MIA-15-5 directed to H/$Le^y$/$Le^b$ inhibited lung metastasis of highly metastatic F10 and BL6 varient of B16 mouse melanoma metastasis in mouse lung. Furthermore, monoclonal antibody FH7 directed to disialosyl-$Le^a$ and monosialosyl-$Le^a$ II inhibited adhesion of human cancer cells expressing these antigens in a dynamic flow system.

Based on these various observations and considerations, this invention provides the following methods:

a) Tumor cell metastasis based on tumor cell adhesion mediated by carbohydrate antigen can be inhibited by such oligosaccharides comprising GM3, H, $Le^y$, $Le^b$, sialosyl-$Le^x$, monosialosyl-$Le^a$ I, monosialosyl-$Le^a$ II, sialosyl Tn, lactosyl and other structures as shown in structures 1-13, in Example 3.

b) Oligosaccharide derivatives based on those structures and linked to an appropriate carrier.

c) Oligosaccharide derivatives whose sugar structures are modified appropriately showing better blocking activity of tumor cell adhesion based on oligosaccharide-lectin (selectin; LECCAM) or oligosaccharide-oligosaccharide interaction).

d) Utilization of antibodies recognizing those oligosaccharides comprising and representing tumor-associated carbohydrate antigens may also inhibit tumor cell adhesion on endothelial cells or platelets or upon target cells, and may inhibit metastasis.

Thus, in one aspect of the present invention, a method for inhibiting tumor cell metastasis potential within a biological preparation is provided. The method comprises incubating the biological preparation with at least one agent selected from the group consisting of (a) tumor-associated carbohydrate antigens that exhibit differential prognostic significance, (b) antibodies that specifically bind to these antigens, (c) oligosaccharide components of these antigens, (d) conjugates of these antigens or oligosaccharides, and (e) mimetics of the tumor-associated carbohydrate antigens, the agent inhibiting the metastasis potential of the preparation. Suitable biological preparations include cell cultures and biological fluids.

Another aspect of the present invention provides a method for inhibiting metastasis potential of tumor cells in a warm-blooded animal. The method comprises administering to a warm-blooded animal an effective amount of at least one agent selected from the group consisting of (a) tumor-associated carbohydrate antigens that exhibit differential prognostic significance, (b) antibodies that specifically bind to these antigens, (c) oligosaccharide components of these antigens, (d) conjugates of these antigens or oligosaccharide components, and (e) mimetics of the tumor-associated carbohydrate antigens, the agent inhibiting tumor cell metastasis potential.

Within a related aspect, the present invention provides a variety of glycoconjugates useful for prolonging the in vivo lifetime of oligosaccharide components. The conjugates comprise an oligosaccharide coupled to poly(ethylene glycol).

Additional oligosaccharide components for use within the methods and compositions of the present invention include lactose, lacto-N-tetrose, methyl β-D-lactoside and phenyl β-D-thiolactoside. Oligosaccharide components may be used individually or in combination with one another.

The present invention further provides a variety of methods for inhibiting GMP-14O-mediated or ELAM-1-mediated cell aggregation or adhesion causing metastasis at a tumor site and inflammatory responses at a site.

One such method inhibits GMP-140-mediated or ELAM-1-mediated cell aggregation or adhesion within a biological preparation .and comprises incubating the biological preparation with at least one agent selected from the group consisting of (a) monosialosyl-$Le^a$ I, monosialosyl-$Le^a$ II, disialosyl-$Le^a$, or sialosyl $Le^x$, (b) antibodies that specifically bind to the monosialosyl-$Le^a$ I, monosialosyl-$Le^a$ II, disialosyl-$Le^a$, or sialosyl $Le^a$, (c) oligosaccharide components of the monosialosyl-$Le^a$ I, monosialosyl-$Le^a$ II, disialosyl-$Le^a$, or sialosyl $Le^x$, (d) conjugates of the monosialosyl-$Le^a$ I, monosialosyl-$Le^a$ II, disialosyl-$Le^a$, or sialosyl $Le^x$ or of the oligosaccharide components, and (e) mimetics of the monosialosyl-$Le^a$ I, monosialosyl-$Le^a$ II, disialosyl-$Le^a$, or sialosyl $Le^x$, said agent inhibiting the cell aggregation or adhesion.

Another such method inhibits GMP-140-mediated or ELAM-1-mediated cell aggregation or adhesion at a tumor cell site in a warm-blooded animal thereby reducing metastatic potential at the site and comprises administering to the warm-blooded animal on effective amount of at least one agent selected from the group consisting of (a) monosialosyl-$Le^a$ I, monosialosyl-$Le^a$ II, disialosyl-$Le^a$, or sialosyl $Le^x$, (b) antibodies that specifically bind to the monosialosyl-$Le^a$ I, monosialosyl-$Le^a$ II, disialosyl-$Le^a$, or sialosyl $Le^x$, (c) oligosaccharide components of the monosialosyl-$Le^a$ I, monosialosyl-$Le^a$ II, disialosyl-$Le^a$, or sialosyl $Le^x$, (d) conjugates of the monosialosyl-$Le^a$ I, monosialosyl-$Le^a$ II, disialosyl-$Le^a$, or sialosyl $Le^x$ or of the oligosaccharide components, and (e) mimetics of the monosialosyl-$Le^a$ I, monosialosyl-$Le^a$ II, disialosyl-$Le^a$, or sialosyl $Le^x$, the agent reducing the metastatic potential at the tumor cell site in the warm-blooded animal.

The present invention also provides a method of inhibiting GMP-140-mediated cell aggregation or adhesion at a site of inflammation in a warm-blooded animal thereby reducing inflammatory potential at the site and comprises administering to warm-blooded animal an effective amount of at least one agent selected from the group consisting of (a) monosialosyl-$Le^a$ I, monosialosyl-$Le^a$ II, disialosyl-$Le^a$, or sialosyl $Le^x$, (b) antibodies that specifically bind to the monosialosyl-$Le^a$ I, monosialosyl-$Le^a$ II, disialosyl-$Le^a$, or sialosyl $Le^x$, (c) oligosaccharide components of the monosialosyl-$Le^a$ I, monosialosyl-$Le^a$ II, disialosyl-$Le^a$, or sialosyl $Le^x$, (d) conjugates of the monosialosyl-$Le^a$ I, monosialosyl-$Le^a$ II, disialosyl-$Le^a$, or sialosyl $Le^x$ or of the oligosaccharide components, and (e) mimetics of the monosialosyl-$Le^a$ I, monosialosyl-$Le^a$ II, disialosyl-$Le^a$, or sialosyl $Le^x$, the agent reducing the inflammatory potential at the inflammatory site in the warm-blooded animal.

In another aspect, the present invention provides a method for identifying a tumor associated carbohydrate antigen (TACA) epitope to which lectin activity of GMP-140 is directed, comprising: (A) constructing a fluorescent probe comprising fluorescent plastic beads coated with the TACA epitope suspected of being targeted by the GMP-140; (B) incubating the fluorescent probe with a suspension of platelets; and (C) determining the degree of binding of the fluorescent probe to the platelets.

These and other aspects of the present invention will become evident upon reference to the following detailed description and attached drawings.

## Brief Description of the Drawings

Figure 1 graphically illustrates the effects of methyl β-D-lactoside or methyl β-D-thiolactoside on the number and size of lung colony deposits of BL6 cells. BL6 were preincubated with control medium, 0.1 M methyl β-D-lactoside ("Me-β-lactoside"), or 0.1 M phenyl β-D-thiolactoside ("phe-β-S-lactoside). 2 x $10^4$ cells were injected intravenously into C57/BL mice. Lung colony numbers were counted at 21 days, and colonies were classified on the basis of diameter (> 1 mm vs. < 1mm), as indicated for each column. Colony numbers are expressed per single lung. Number of experiments ("n") is indicated in parentheses.

Figure 2 graphically illustrates the effect of prior administration of methyl β-D-lactoside on the number and size of lung colony deposits of BL6 cells. Methyl β-D-lactoside (1 ml dosage) was injected intraperitoneally into C57/BL mice. After 10 minutes, B16 melanoma cells were injected intravenously. Lung colonies were counted and sized at 19 days. Group A represents control animals (not administered methyl β-D-lactoside) and groups B and C represent animals injected with 0.25 M and 0.5 M methyl β-D-lactoside, respectively. For each group, column 1 represents the total number of colonies, column 2 the number of colonies with diameter > 1 mm, and column 3 the number of colonies with diameter < 1 mm. Number of experiments is expressed as "n".

Figure 3 graphically illustrates survival of cancer patients with or without expression of a defined tumor associated carbohydrate antigen (TACA) in their tumors. Panel A represents the expression of H/$Le^y$/$Le^b$ antigen in lung squamous cell carcinoma as determined by monoclonal antibody MIA-15-5. Panel B represents sialosyl-$Le^x$ expression in colonic cancer. Panel C represents sialosyl-Tn expression in colonic cancer. Panel D represents sialosyl-Tn level in sera of ovarian cancer patients.

Figure 4 graphically illustrates that melanoma cell adhesion on LacCer is based on GM3-LacCer interaction. The order of metastatic potential is BL6>F10>F1>>WA4. Panel A shows the order of melanoma cell adhesion on LacCer-coated solid phase. Panel B shows the order of melanoma cell adhesion on LacCer/Fibronectin (FN) co-coated solid phase. Panel C shows integrin-dependent adhesion.

Figure 5 graphically illustrates the melanoma cell (BL6) adhesion on LacCer (Panel A) and on endothelial cells (HuVEC) (Panel B) are inhibited by LacCer and GM3.

Figure 6 graphically illustrates the metastasis-inhibiting effect of methyl(Me)-β-lactoside. Tumor cells were intravenously injected, followed by intraperitoneal injection of: PBS control (A); 0.25 M Me-β-lactoside (B); 0.5 M Me-β-lactoside; (C); 0.5 M lactose (D); 0.25 M N-acetyllactosamine (E); or 0.5 M Me-β-galactoside (E).

Figure 7 graphically illustrates H-$Le^y$ and H-H interaction. Panel A shows H1-liposome binding to various glycolipids. Panel B shows $Le^y$-liposome binding to various glycolipids.

Figures 8A-8D are flow cytometric profiles of non-activated (Fig. 8A and Fig. 8C) and activated (Fig. 8B and Fig. 8D) platelets with anti-GMP-140 phase monoclonal antibody.

Figure 9 graphically illustrates the binding indexes of platelets with fluorescent beads coated with various GSLs. The hatched bars represent non-activated platelets and the open bars represent activated platelets.

Figure 10 graphically illustrates the effects of various monoclonal antibodies on binding of activated platelets to sialosyl-$Le^a$ coated beads. The abscissa represents the percent inhibition. Column 1 represents anti-GMP-140-MAb-IOP62; Column 2 represents anti-sialosyl-$Le^a$ monoclonal antibody CA19-9; Column 3 represents anti-sialosyl-$Le^x$ monoclonal antibody SNH4; and Column 4 represents normal mouse IgG.

Figures 11A-11D illustrate experimental systems demonstrating dynamic adhesion of cells in a flow system. Fig. 11A shows the structure of the laminar flow chamber. Fig. 11B indicates a cross section pattern of a laminar chamber in which flow chamber body (16) is tightly affixed with the cover slip (3) on which cells or adhesion molecules (9) are fixed. Fig. 11C shows the entire assembly of the recording system. Fig. 11D is a schematic presentation of the flow of tumor cells in suspension passing over the cell layer or adhesion molecule.

Figure 12 is a graph showing the effect of various monoclonal antibodies on adhesion of human colon carcinoma Colo205 cells to interleukin-1-activated human umbilical vein endothelial cells in a dynamic flow system. In the figure, the open circles represent a mixture of irrelevant mouse IgG plus IgM (control), the solid triangles represent monoclonal antibody CA19-9 directed to monosialosyl-$Le^a$ I, the open triangles represent monoclonal antibody SNH4 directed to sialosyl-$Le^x$, the solid circles represent monoclonal antibody FH7 directed to monosialosyl-$Le^a$ II and disialosyl-$Le^a$, and the solid squares represent a mixture irrelevant mouse IgG plus IgM and non-activated endothelial cells.

## Detailed Description of the Invention

As noted above, the present invention in one aspect is directed toward methods and compositions for the inhibition of tumor cell metastasis potential and invasiveness. Numerous tumor cells possess the ability to metastasize, i.e., to form a secondary tumor colony at a distant site. Sources of malignant tumor cells include melanoma, lung, breast, colorectal and urogenital cancers, such as bladder and prostate cancers. Within the present invention, the metastasis potential of tumor cells, (i.e., inhibiting the ability of tumor cells to metastasize) may be inhibited through the use of (a) tumor-associated carbohydrate antigens (TACAs); (b) antibodies directed to these TACAs; (c) oligosaccharide components of these TACAs; (d) conjugates of such TACAs or of oligosaccharide components of such TACAs, such as multivalent conjugates of lysyllysine or TACA-bearing glycosphingolipid (GSL) liposomes; or (e) mimetics of the TACAs.

TACA epitopes play essential roles in tumor cell adhesion through their interaction with endothelial cells, platelets and basement membranes, whereby tumor metastasis and invasion may occur. The mechanism of adhesion may be based upon carbohydrate (CHO)-CHO interaction, CHO-lectin interaction or selectin family interaction. Adhesion of various tumor cells on activated endothelial cells and platelets is mediated primarily by the LECCAM or selectin superfamily (e.g., ELAM-1, GMP-140). Tumor cell adhesion mediated by sialosyl-$Le^x$ is inhibited by anti-sialosyl-$Le^x$ monoclonal antibodies (FH6, CSLEX, SNH3, SNH4) and tumor cell adhesion mediated by monosialosyl-$Le^a$ I is inhibited by monoclonal antibodies (CA19-9, CSLEA, NKH1, NKH2) directed to this epitope. Also, Colo205 tumor cells, which express predominantly type 1 chain sialosyl-$Le^a$ and to a lesser extent sialosyl-$Le^x$, adhere to endothelial cells. This adhesion was inhibited by anti-sialosyl-$Le^a$ monoclonal antibody and to a lesser extent by anti-sialosyl-$Le^x$ monoclonal antibody. These findings suggest that not only sialosyl-$Le^x$, but also sialosyl-$Le^a$, are the important ligands recognized by ELAM-1 and GMP-140 (previously termed CD62 or PADGEM). The present inventors have also found that adhesion of tumor cells on activated endothelial cells is also based on recognition of monosialosyl-$Le^a$ II and disialosyl-$Le^a$. Both monosialosyl-$Le^a$ II and disialosyl-$Le^a$ are defined by monoclonal antibody FH7, which is known to strongly inhibit adhesion of various types of epithelial cancer cells (particularly colorectal, gastrointestinal, and pancreatic) to activated endothelial cells or platelets via selections.

In particular, GMP-140 is the major selectin (LECCAM) located on α-granules of platelets, or Weibel-Palade bodies of endothelial cells (ECs). On activation of these cells, GMP-140 is rapidly redistributed to the cell surface, where it plays an important role in adhesion of platelets or ECs to certain carbohydrate epitopes expressed on blood cells or tumor cells, resulting in aggregation of platelets or tumor cells, or their adhesion to capillary endothelia. GMP-140-mediated cell adhesion is believed by the present inventors to be involved in initiation of metastatic deposition of tumor cells and initiation of inflammatory processes.

Also, ELAM-1 is expressed on endothelial cells after activation with interleukin-1, TGF-β, TNF-α, or lipopolysaccharide. ELAM-1-mediated cell adhesion is also believed by the present inventors to be involved in initiation of metastatic deposition of tumor cells.

Thus, the present invention in another aspect is directed towards inhibiting GMP-140-mediated or ELAM-1-mediated cell aggregation or adhesion, especially at tumor cell sites. Within the present invention, GMP-140-mediated cell aggregation or adhesion can be inhibited through the use of (a) the TACA monosialosyl-$Le^a$ I, monosialosyl-$Le^a$ II, disialosyl-$Le^a$, or sialosyl $Le^x$ (b) antibodies that specifically bind to monosialosyl-$Le^a$ I, monosialosyl-$Le^a$ II, disialosyl-$Le^a$, or sialosyl $Le^x$, (c) oligosaccharide components of the monosialosyl-$Le^a$ I, monosialosyl-$Le^a$ II, disialosyl-$Le^a$, or sialosyl $Le^x$, (d) conjugates of the monosialosyl-$Le^a$ I, monosialosyl-$Le^a$ II, disialosyl-$Le^a$, or sialosyl $Le^x$ or of the oligosaccharide components, and (e) mimetics of the monosialosyl-$Le^a$ I, monosialosyl-$Le^a$ II, disialosyl-$Le^a$, or sialosyl $Le^x$.

Within the present invention, tumor metastasis and invasion is inhibited by blocking tumor cell adhesion, thereby significantly reducing or eliminating the spread of metastatic cells.

Also within the present invention, tumor metastasis and invasion is inhibited by inhibiting: (1) GMP-140-mediated tumor cell aggregation or adhesion at a tumor site due to: (a) adhesion of tumor cells to platelets, (b) adhesion of tumor cells to tumor cells via platelets, (c) adhesion of tumor cells to ECs via platelets, and (d) adhesion of tumor cells to ECs directly via GMP-140; and (2) ELAM-1-mediated tumor cell aggregation or adhesion at a tumor site due to adhesion of cells to ECs directly via ELAM-1.

Further within the present invention, inflammation is inhibited by inhibiting GMP-140-mediated cell aggregation or adhesion at a potential site of inflammation due to: (a) adhesion of cells to platelets, (b) adhesion of cells to cells via platelets, (c) adhesion of cells to ECs via platelets, and (d) adhesion of cells to ECs directly via GMP-140.

TACAs suitable for use within the present invention are those showing differential prognostic significance (i.e., TACAs that may be clearly correlated with invasive or metastatic potential). Within the context of the present invention, such TACAs may be distinguished through a comparison of invasiveness, metastasis and clinical prognosis of similar tumors showing expression vs. non-expression of such TACAs. Preferred TACAs for use within the present invention include H/$Le^y$/$Le^b$, sialosyl-$Le^x$ (SA-$Le^x$), monosialosyl-$Le^a$ I (SA-$Le^a$), and sialosyl-Tn (SA-Tn). Derivatives of such TACAs include dimeric $Le^x$, sialosyl-dimeric $Le^x$, trifuscosyl $Le^y$, disialosyl-$Le^a$, and monosialosyl-$Le^a$ II.

As noted above, TACAs for use within the present invention exhibit a differential prognostic significance. By way of example, such a differential prognostic significance may be illustrated by the fact that tumors expressing H/$Le^y$/$Le^b$ antigens (as defined by monoclonal antibody MIA-15-5) showed much worse patient prognosis than tumors not expressing these antigens. For instance, as shown in Figure 3A, patients with squamous cell lung carcinoma expressing H/$Le^y$/$Le^b$ had only an 11% survival over a 5-year period (i.e., 89% died), whereas comparable patients not expressing H/$Le^y$/$Le^b$ had an approximately 62% survival over this period. Similar results were obtained for tumors showing expression vs. non-expression of sialosyl-$Le^x$ and sialosyl-Tn antigens. More specifically, as shown in Figure 3B, patients with colonic cancer expressing sialosyl-$Le^x$ had only a 15% survival over a 5-year period, whereas comparable patients not expressing this antigen had an approximately 50% survival over this period. In a separate study, the 5-year survival of patients with early-stage colonic cancer not expressing sialosyl-Tn was 100%, as compared to 75% for patients who expressed sialosyl-Tn (see Figure 3C). As shown in Figure 3D, similar but more obvious differences were observed in patients with ovarian cancer showing expression vs. non-expression of sialosyl-Tn antigen.

Also as noted above, antibodies to suitable TACAs may be employed within the context of the present invention. As used herein, such antibodies include both monoclonal and polyclonal antibodies and may be intact molecules, a fragment of such a molecule, or a functional equivalent thereof. The antibody may be genetically engineered. Examples of antibody fragments include $F(ab')_2$, Fab′, Fab and Fv.

Briefly, polyclonal antibodies may be produced by immunization of an animal and subsequent collection of its sera. Immunization is accomplished, for example, by a systemic administration, such as by subcutaneous, intrasplenic or intramuscular injection, into a rabbit, rat or mouse. It is generally preferred to follow the initial immunization with one or more booster immunizations prior to sera collection. Such methodology is well known and described in a number of references.

While polyclonal antibodies may be employed in the present invention, monoclonal antibodies are preferred. monoclonal antibodies suitable for use within the present invention include those of murine or human origin, or chimeric antibodies such as those which combine portions of both human and murine antibodies (i.e., antigen binding region of murine antibody plus constant regions of human antibody). Human and chimeric antibodies

may be produced using methods known by those skilled in the art. Human antibodies and chimeric human-mouse antibodies are advantageous because they are less likely than murine antibodies to cause the production of anti-antibodies when administered clinically.

Monoclonal antibodies may be generally produced by the method of Köhler and Milstein (Nature 256:495-497, 1975; Eur. J. Immunol. 6:511-519, 1976), as well as by various techniques which modify Köhler and Milstein's initial method (see Harlow and Lane (eds.), Antibodies: A Laboratory Manual, Cold Spring Harbor Laboratory, 1988, which is herein incorporated by reference in its entirety). Briefly the lymph nodes and/or spleens of an animal immunized with one of the TACAs or their oligosaccharide components are fused with myeloma cells to form hybrid cell lines ("hybridomas" or "clones"). Each hybridoma secretes a single type of immunoglobulin and, like the myeloma cells, has the potential for indefinite cell division. It may be desirable to couple such molecules to a carrier to increase their immunogenicity. Suitable carriers include keyhole limpet hemocyanin, thyroglobulin, bovine serum albumin and derivatives thereof. An alternative to the production of monoclonal antibodies via hydridomas is the creation of monoclonal antibodies expression libraries using bacteriophage and bacteria (e.g., Sastry et al., Proc. Natl, Acad. Sci USA 86:5728, 1989; Huse et al., Science 246:1275, 1289). Selection of antibodies exhibiting appropriate specificity may be performed in a variety of ways which will be evident to those skilled in the art.

Representative examples of monoclonal antibodies suitable for use within the present invention include MIA-15-5 (Miyake and Hakomori, Biochem. 30:3328, 1991), as well as the monoclonal antibodies cited within Hakomori, Advances In Cancer Research 52:257-331, 1989.

As discussed above, oligosaccharide components of suitable TACAs may also be used within the present invention. As used herein, the term "oligosaccharide" includes naturally derived oligosaccharides, synthetically prepared, and derivatives of either, including portions of a TACA oligosaccharide component.

Additional oligosaccharide components useful within the present invention include lactose and lactose derivatives, such as methyl β-D-lactoside, lact-N-tetrose (Ga1β1→3G1cNAcβ1→3Ga1β1→4G1c), and phenyl β-D-thiolactoside. Other lactose derivatives may also be used, including ethyl or phenyl lactoside and methyl or ethyl thiolactoside.

Other oligosaccharide components suitable for inhibiting metastasis potential of cells of a particular tumor may be identified based upon determination of the structure of specific carbohydrate chain(s) which are involved in the tumor's ability to metastasize. The identification of carbohydrate-containing molecules involved in a tumor's ability to metastasize may be accomplished in a variety of ways, including through the use of glycosidases and inhibitors of glycosyltransferases. The structure of carbohydrates bound to either lipids or proteins may be determined based on degradation, mass spectrometry, including electron-impact direct-probe (EI) and fast atom bombardment (FAB), and methylation analysis (techniques described, for example, in Nudelman et al., J. Biol. Chem. 261:5487-5495, 1986). Degradation analysis may be accomplished chemically and/or enzymatically, e.g., by glycosidases. The carbohydrate sequence suggested by degradation analysis may be determined by methylation analysis (Hakomori, J. Biochem. 55:205-208, 1964) followed by chemical ionization mass spectrometry of permethylated sugars (Stellner et al., Arch Biochem. Biophys. 155:464-472, 1974; Levery et al., Meth. Enzymol. 138:13-25, 1987). Alternatively, or in conjunction with these techniques, EI mass spectrometry may be performed on permethylated glycans or after the appropriate degradation of intact glycans (Kannagi et al., J. Biol. Chem. 259:8444-8451, 1984; Nudelman et al., J. Biol. Chem. 263:13942-13951, 1988). Homogeneity of the carbohydrate sequence may be demonstrated based on various chemical and physical criteria, including proton NMR spectroscopy of intact or methylated glycans and FAB mass spectrometry. Once the carbohydrate sequence has been determined, it will be evident to those of ordinary skill in the art to select an appropriate oligosaccharide for inhibiting the tumor cell's metastasis potential.

As briefly discussed above, conjugates of suitable TACAs or oligosaccharide components thereof, such as multivalent conjugates with lysyllysine or TACA-bearing glycosphingolipid (GSL) liposomes, may also be used within the present invention.

The components of the conjugate may be covalently coupled to one another either directly or via a linker group. A direct reaction between components is possible when each possesses a substituent capable of reacting with the other. For example, a nucleophilic group, such as an amino or sulfhydryl group, on one component may be capable of reacting with a carbonyl-containing group, such as anhydride or an acyl halide, or with an alkyl group containing a good leaving group, e.g., a halide, on the other.

It may be desirable to covalently couple components via a linker group. A linker group can serve to increase the chemical reactivity of a substituent, and thus increase the coupling efficiency. An increase in chemical reactivity may also facilitate the use of functional groups on components which would not otherwise be possible. For example, a carboxyl group may be activated. Activation of a carboxyl group includes formation of an "active ester", such as a succinimidyl ester. The term "active ester" is known to refer to esters which are highly reactive in nucleophilic substitution reactions.

Alternatively, it may be desirable to produce conjugates in which the components are non-covalently linked. For example, one or more TACAs may be incorporated into the outer surface of GSL liposomes.

It may be desirable to increase the *in vivo* lifetime of an oligosaccharide. As disclosed within the present invention, oligosaccharides may be coupled to (i.e., covalently bonded to) a straight-chain amphophilic polymer, such as poly(ethylene glycol). A representative example of a method for producing an oligosaccharide-poly(ethylene glycol) conjugate is the reaction of an oligosaccharide, which has been derivatized to contain a succinimidyl group, with a poly(ethylene glycol) having a terminal amino group. The latter compound has a general formula of $NH_2$-$(CH_2CH_2$-$O)_n$-$CH_3$, where n typically averages 44.7 (i.e., molecular weight of about 2,000) to 112.9 (i.e., molecular weight of about 5,000).

Additionally, because the cell adhesion mediated by selectins, ELAM-1 or GMP-140, is based on recognition of sialylated and fucosylated lactoseries type 1 and type 2 chains by a lectin sequence domain present at the N-terminal region of the selectin molecules, any structures which may show more effective blocking activity of the lectin domain than naturally occurring epitopes are useful in the present invention. Such unnatural synthetic compounds, termed "mimetics", of, for example, sialosyl-$Le^x$ or sialosyl-$Le^a$ I or II, which mimic the surface structure of naturally occurring epitopes but show better blocking activity of carbohydrate-dependent adhesion can be considered. Examples of useful mimetics include, but are not limited to, sialosyl-$Le^x$ or monosialosyl-$Le^a$ I or II having trifluoro-L-fucose, N-trifluoro-acetyl-glucosamine or a heterocyclic or aromatic ring structure having a sialic acid analog and fucose analog at the same distance and spacial configuration as those found in naturally occurring sialosyl-$Le^x$, monosialosyl-$Le^a$ I and II, or the H/$Le^y$/$Le^b$ structure having trifluoro-L-fucose, N-trifluoro-acetyl-glucosamine, or sialosyl-Tn analogs containing N-trifluoro-acetyl-neuraminic acid. Thus, a modified carbohydrate epitope, or any other "mimetic" mimicking the surface structure of a carbohydrate epitope, which blocks cell adhesion through tumor-associated carbohydrates more efficiently than a naturally occurring epitope is within the present invention.

The inhibition of metastasis potential of tumor cells and GMP-140-mediated or ELAM-1-mediated cell aggregation or adhesion have a variety of *in vitro* and *in vivo* uses, e.g., treatment of isolated tumor cells or tumor-bearing hosts and treatment of disease processes involving GMP-14O or ELAM-1.

Regarding *in vitro* aspects, as noted above, the present invention provides a method for inhibiting tumor cell metastasis potential within a biological preparation. The method comprises incubating a biological preparation with at least one agent selected from the group consisting of (a) tumor-associated carbohydrate antigens that exhibit differential prognostic significance, (b) antibodies that specifically bind to these antigens, (c) oligosaccharide components of these antigens, (d) conjugates of these antigens or oligosaccharide components, and (e) mimetics of the tumor-associated carbohydrate antigens, the agent inhibiting the metastasis potential of the preparation.

Regarding further *in vitro* aspects, the present invention also provides a method for inhibiting GMP-140-mediated or ELAM-1-mediated cell aggregation or adhesion within a biological preparation. This method comprises incubating the biological preparation with at least one agent selected from the group consisting of (a) monosialosyl-$Le^a$ I, monosialosyl-$Le^a$ II, disialosyl-$Le^a$, or sialosyl $Le^x$, (b) antibodies that specifically bind to the monosialosyl-$Le^a$ I, monosialosyl-$Le^a$ II, disialosyl-$Le^a$, or sialosyl $Le^x$, (c) oligosaccharide components of the monosialosyl-$Le^a$ I monosialosyl-$Le^a$ II, disialosyl-$Le^a$, or sialosyl, (d) conjugates of the monosialosyl-$Le^a$ I, monosialosyl-$Le^a$ II, disialosyl-$Le^a$, or sialosyl $Le^x$ or of the oligosaccharide components, and (e) mimetics of the monosialosyl-$Le^a$ I, monosialosyl-$Le^a$ II, disialosyl-$Le^a$, or sialosyl $Le^x$, the agent inhibiting the cell aggregation or adhesion.

Suitable biological preparations include cell cultures and cell suspensions in biological fluids, such as blood, urine, lymph, synovial and cerebrospinal fluid. TACAs, oligosaccharides or conjugates thereof will generally be incubated at a final concentration of about 0.1 to 1 M, and typically at about 0.2 to 0.5 M. Incubation is typically performed for 5 to 15 minutes at 37°C. After treatment of a biological preparation, the preparation may be injected or implanted in an animal, e.g., to confirm effectiveness of the inhibition of metastasis potential.

The present invention also provides a method for inhibiting tumor cell metastasis potential in a warm-blooded animal, such as a human. The method comprises administering to a warm-blooded animal an effective amount of at least one agent selected from the group consisting of (a) tumor-associated carbohydrate antigens that exhibit differential prognostic significance, (b) antibodies that specifically bind to these antigens, (c) oligosaccharide components of these antigens, (d) conjugates of these antigens or the oligosaccharide components, and (e) mimetics of the monosialosyl-$Le^a$ I, monosialosyl-$Le^a$ II, disialosyl-$Le^a$, or sialosyl $Le^x$, the agent inhibiting the metastasis potential of the preparation.

Similarly, the present invention also provides a method for inhibiting GMP-140-mediated or ELAM-1-mediated cell aggregation or adhesion at a tumor cell site in a warm-blooded animal. The method comprises administering to a warm-blooded animal an effective amount of at least one agent selected from the group consisting of (a) monosialosyl-$Le^a$ I, monosialosyl-$Le^a$ II, disialosyl-$Le^a$, or sialosyl $Le^x$, (b) antibodies that specifically bind to monosialosyl-$Le^a$ I, monosialosyl-$Le^a$ II, disialosyl-$Le^a$, or sialosyl $Le^x$, (c) oligosaccharide components of the monosialosyl-$Le^a$ I, monosialosyl-$Le^a$ II, disialosyl-$Le^a$, or sialosyl $Le^x$, (d) conjugates of the monosialosyl-$Le^a$ I, monosialosyl-$Le^a$ II, disialosyl-$Le^a$, or sialosyl $Le^x$ or of the oligosaccharide components, and (e) mimetics of the monosialosyl-$Le^a$ I, monosialosyl-$Le^a$ II, disialosyl-$Le^a$, or sialosyl $Le^x$, said agent reducing the metastatic potential at the tumor site in the warm-blooded animal.

The present invention also provides a method for inhibiting GMP-140-mediated cell aggregation or adhesion at an inflammation site in a warm-blooded animal.

The method comprises administering to warm-blooded animal an effective amount of at least one agent selected from the group consisting of (a) monosialosyl-$Le^a$ I, monosialosyl-$Le^a$ II, disialosyl-$Le^a$, or sialosyl $Le^x$, (b) antibodies that specifically bind to the monosialosyl-$Le^a$ I, monosialosyl-$Le^a$ II, disialosyl-$Le^a$ , or sialosyl $Le^x$, (c) oligosaccharide components of the monosialosyl-$Le^a$ I, monosialosyl-$Le^a$ II, disialosyl-$Le^a$, or sialosyl $Le^x$, (d) conjugates of the monosialosyl-$Le^a$ I, monosialosyl-$Le^a$ II, disialosyl-$Le^a$, or sialosyl $Le^x$ or of the oligosaccharide components, and (e) mimetics of the monosialosyl-$Le^a$ I, monosialosyl-$Le^a$ II, disialosyl-$Le^a$, or sialosyl $Le^x$, the agent reducing the inflammatory potential at the inflammatory site in the warm-blooded animal.

For both methods, TACAS, oligosaccharides or conjugates thereof will generally be administered at a concentration of about 0.1 to 1 M and typically at about 0.2 to 0.5 M. It will be evident to those skilled in the art how to determine the optimal effective dose for a particular substance, e.g., based upon *in vitro* and *in vivo* studies in non-human animals. A variety of routes of administration may be used. Typically, administration will be intravenous or intracavitary, e.g., in pleural or peritoneal cavities, in the bed of a resected tumor or at a site of inflammation. A TACA, antibody, oligosaccharide or derivative as discussed above may be administered in combination with a pharmaceutically acceptable carrier or diluent, such as physiological saline. Moreover, the agents that inhibit or reduce metastatic potential may be administered in combination with an immunotherapeutic or chemotherapeutic substance, and the agents that reduce inflammatory potential may be administered in combination with an anti-inflammatory substance. When a combination of such an agent and a substance is desired, each compound may be administered sequentially, simultaneously, or combined and administered as a single composition. Diagnostic techniques, such as CAT scans, may be performed prior to and subsequent to administration to confirm the effectiveness of the inhibition of metastatic potential or inflammatory potential.

One in vitro system for measuring adhesion or aggregation of tumor cells to other cells (e.g. ECs), or for determining successful inhibition of the adhesion or aggregation is a dynamic flow system similar to that described by M. B. Lawrence et al. (Blood 70:1284, 1987) and which is shown in Figures 11A, 11B, 11C, and 11D.

A parallel-plate laminar flow chamber **(1)** (shown upside down for convenience) connected to a pressure pump **(2)** via tubing **(18)** is used to simulate the flow shear stresses present in physiological microvascular environments. The flow chamber consists of a plastic or glass cover slip **(3)** resting on a chamber body **(16)** on which a parallel, transparent plastic surface **(4)** is attached with a rubber or silicone gasket **(5)**; there is a 114 μm gap between the two surfaces, and this gap is connected to an inlet slot **(6)** connected to an inlet manifold **(8)** and outlet slot **(7)** connected to an outlet manifold **(19)** (Fig. 11A). A laminar flow with defined rate and wall shear stress is achieved by manipulation of the pressure pump **(2)**, which is connected to the inlet manifold **(8)** of the flow chamber via tubing **(18)**. Fig. IIB shows the assembled flow chamber **(1)**.

In order to fix together the cover slip **(3)** and the chamber body **(16)** very tightly, there is a continuous circular grooved space **(20)** on the periphery of the chamber body **(16)**. This circular, grooved space connects to a vaccum pump by placement of the rubber or silicone rubber gasket **(5)** with cover slip **(3)** on top. Thus, by applying vacccum **(21)** in **(20)** the cover slip **(3)** and chamber body **(16)** are strongly affixed and immovable. The thin inlet and outlet slots in the chamber body **(16)** open to the inlet and outlet manifolds respectively. The outlet manifold is connected to a pressure pump **(2)** which can be operated in either a negative or positive way.

Cells (e.g., endothelial cells) are grown on either a glass or plastic cover slip **(3)**, and a tumor cell suspension in medium flows from inlet manifold **(16)** to outlet manifold **(19)**. The structure of the flow chamber **(1)** in Figure 11B is shown upside down for convenience. This chamber is placed under an inverted microscope stage, right side up (Fig. 11C), and the flow of tumor cells over the cell layer (e.g., endothelial cell layer) is observed under the microscope. The observed pattern of rolling and stopping (i.e., pattern of adhesion) of tumor cells can be recorded on videotape.

Turning to Fig. 11C, the cells **(9)** are grown as a monolayer on the cover slip **(3)**, and a laminar flow of tumor cell suspension **(14)**, maintained in a vessel in a water bath **(17)**, is passed through the chamber via tubing **(18)**. Cell movements are observed under an inverted phase-contrast microscope **(10)** and recorded by time-lapse videocassette recorder **(11)** using a video camera **(12)** and a digital image processor**(13)**. Adhesion is observed as rolling followed by stopping of cells. Number of cells bound during a set time, e.g. 3 minutes, at different shear stresses, e.g., from 0.4 to 4.8 dynes/$cm^2$, are counted from several fields recorded on videotape (Fig. 11B). Wall shear stress (T) is calculated as $3\mu Q/2ba^2$, where $\mu$ = coefficient of viscosity, e.g. 1.0 cP, Q = volumetric flow rate ($cm^3$/sec), a = half channel height, e.g. 5.7 x $10^{-3}$ cm, and b = channel width, e.g. 1.3 cm.

Fig. 11D schematically shows laminar flow of tumor cell suspension **(14)** through a chamber in which one surface is coated with endothelial cells **(9)**. Rolling or stopped cells **(15)** are observed under inverted microscope and recorded on videotape as described above. The arrows indicate the direction of flow of the tumor cell suspension **(14)**.

As mentioned above, the present invention also provides a method for identifying a TACA epitope to which lectin activity of the selectin GMP-140 is directed.

Previously, these TACA epitopes were studied based on the inhibitory effect of various glycosphingolipids (GSLs), GSL oligosaccharides, or GSL-containing liposomes on adhesion of blood cells or tumor cells to solid phase (i.e., plastic surface) coated with activated platelets. In practice, this meant coating solid phase with gelatin, which was in turn coated with activated platelets; platelets bind readily to gelatin-coated solid phase via GpIIb/IIIa, the major platelet integrin receptor. In studies using this method, binding of promyelocytic leukemia HL60 cells to platelet-coated solid phase was inhibited by liposomes containing a sialosyl-$Le^x$ determinant, but not by liposomes containing sialosylparagloboside (SPG), and only weakly by liposomes containing $\alpha 1 \rightarrow 3$ fucosylated type 2 chain ($Le^x$) (see Table 1 in Example 3 below). These results suggested that sialosyl-$Le^x$ is the carbohydrate epitope defined by GMP-140 (Polley et al., Proc. Natl. Adac. Sci USA 88: 6224-6228, 1991).

However, the method described above had an important limitation: no cell line which expresses exclusively type 1 chain GSL is available. Myelogenous cell line HL60 and monocytic cell line U937 express exclusively type 2 chain, but not type 1 chain. Essentially all known human tumor cell lines derived from colonic, gastric, or lung carcinoma express both type 1 and type 2 chains. For these reasons, it is difficult to determine the real epitope structure to which GMP-140 binds. To address this problem, a new methodology was developed as described below.

Fluorescent plastic (e.g. polystyrene) beads (diameter $\approx$ 0.5 $\mu$m) are coated with GSL. GSLs are known to be strongly adsorbed on such beads, which allows construction of fluorescent probes containing specific GSLs. Platelets (activated or non-activated) are incubated with such GSL-coated beads, followed by determination of platelet fluorescence intensity by flow cytometry.

Using this method, activated platelets were found to show much stronger binding to fluorescent beads coated with monosialosyl-$Le^a$ I (see Table 1) than to beads coated with any related GSL. The binding of platelets to sialosyl-$Le^a$-coated beads was inhibited by anti-GMP-140 monoclonal antibody, or anti-sialosyl-$Le^a$ monoclonal antibody, but not by anti-sialosyl-$Le^x$ monoclonal antibody. Although binding of activated platelets to sialosyl-$Le^x$-coated beads was observable, it was much lower than binding to sialosyl-$Le^a$-coated beads. These results indicate that the primary epitope structure defined by GMP-140 is sialosyl-$Le^a$, rather than sialosyl-$Le^x$.

Of course, other epitope structures defined by GMP-140 can be identified by use of this present inventive method.

The following examples are offered by way of illustration and not by way of limitation.

## EXAMPLES

### Example 1

### SYNTHESIS OF LACTOSE DERIVATIVES

#### A. Methyl β-D-lactoside

Heptaacetyllactosylimidate (Zimmermann et al., J.Carbohydr. Chem. 7:435, 1988) was reacted with methanol in dry dichloromethane containing trimethylsilyl trifluoromethanesulfonate according to standard procedure (Grundler and Schmit, Liebigs Ann. Chem. 1984:1826, 1984). Purification by silica-gel column chromatography (toluene/EtOAc 1:1 by vol.), followed by de-O-acetylation with 0.01 M sodium methoxide, gave methyl β-D-lactoside in 68% yield from the imidate: m.p. 211-212°C (lit. 205°C, Smith and van Cleve, J. Am. Chem. Soc. 77:3159, 1955); $[\alpha]_D$ + 1.3° (c 6.9, $H_2O$) (lit. + 1°,c 5.0, $H_2O$), ibid).

#### B. Phenyl β-D-thiolactoside

Lactose octaacetate (Hudson and Kunz, J. Am. Chem Soc. 47:2052, 1926) was treated with thiophenol and $SnCl_4$ (Nicolaou et al., J. Am. Chem Soc. 110:7910, 1988) in dichloromethane at 0°C to give phenyl heptaacetyl β-D-thiolactoside in 80% yield. This product was deacetylated with NaOMe in MeOH and neutralized with Amberlyst® 15. Purification of the product on a BioGel® P-2 column using water as an eluent, followed by lyophilization of the sugar-containing fraction, left phenyl β-D-thiolactoside as a white amorphous powder.

C. Lacto-N-tetrose

This oligosaccharide (Galβ1→3GlcNAcβl→3Galβl→4Glc) was prepared from human milk by pretreatment with ethanol and recycling BioGel P-2 column chromatography with water as eluent followed by reversed-phase ($C_{18}$) high pressure liquid chromatography with water (Dua and Bush, Anal. Biochem. 133:1, 1983). The $^1$H-NMR spectrum was superimposed on that of the authentic sample (BioCarb Chemicals, Lund, Sweden).

D. The Poly(ethylene glycol) derivative of β-D-lactoside

The reaction scheme is as follows:

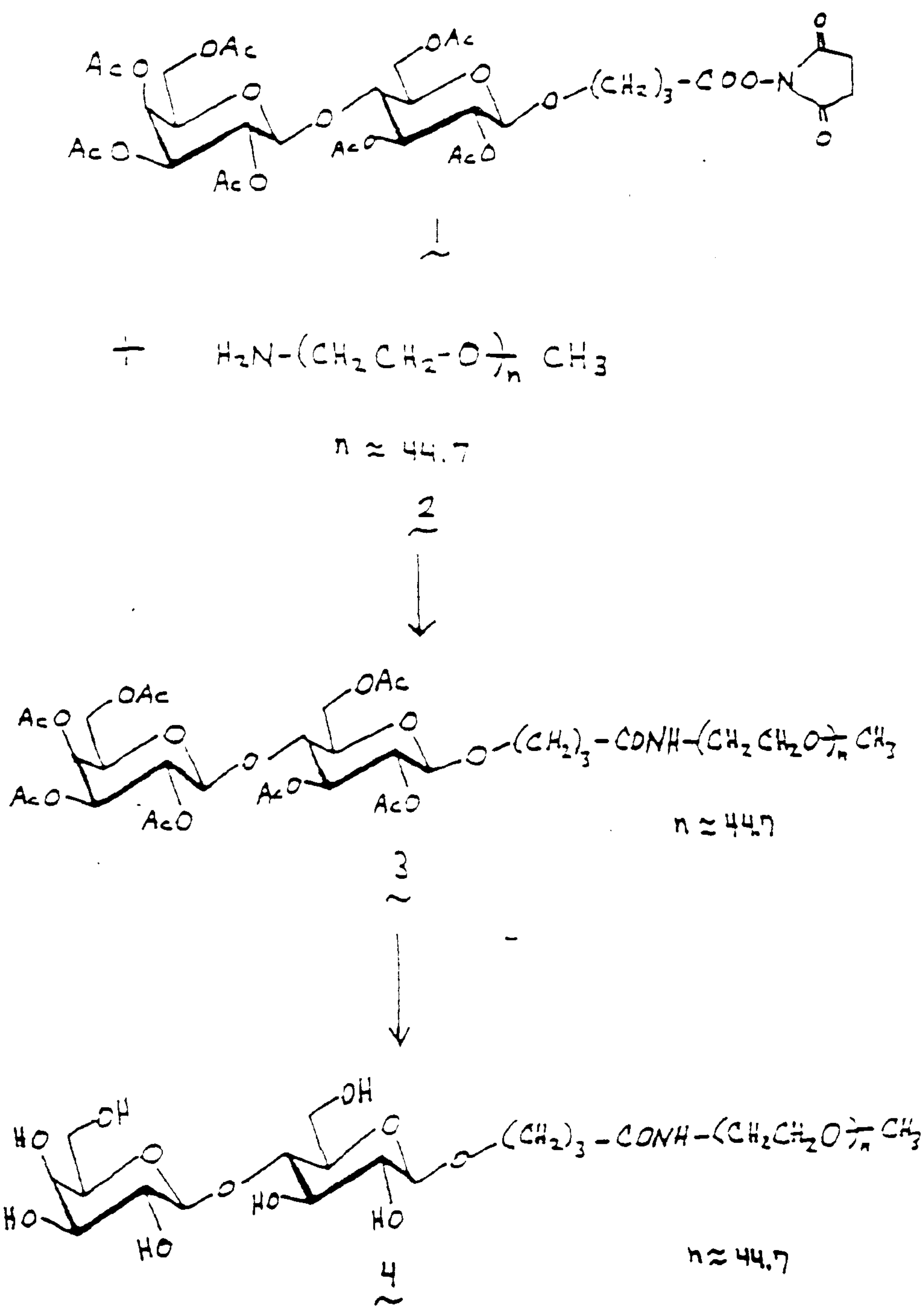


The Poly(ethylene glycol) derivative of β-D-lactoside was prepared from readily available 3-succinimidooxycarbonylpropyl O-(2, 3, 4, 6-tetra-O-acetyl-O-β-D-galactopyranosyl)-(1→4)-2,3,6-tri-O-acetyl-β-D-glucopyranoside 1 and poly(ethylene glycol) methyl ether (average M.W. 2000; Aldrich Chemical, Milwaukee, WI) having a terminal amino group 2 (Zalipsky et al., Eur, Polym, J. 19:1177, 1983). Treatment of 1 (100 mg, 0.12 mmol) and 2 (163 mg, 0.082 mmol) in dry N,N-dimethylformamide (2 mL) at room temperature for 2 hours gave, after chromatography on LH-20 with acetone as an eluent, the β-D-lactoside heptaacetate 3 in 91% yield : $[\alpha]_D$-5.3° (c 0.5, chloroform). A subsequent saponification of 3 with 0.05 M sodium hydroxide at room temperature for one hour, followed by lyophilization, afforded the desired lactoside 4 quantitatively: $[\alpha]_D$-2.4°(c 1.0, chloroform).

Example 2

EFFECT OF LACTOSE AND LACTOSE DERIVATIVES ON METASTATIC POTENTIAL OF B16 MELANOMA CELLS

A. Cells and Animals

The highly metastatic BL6 clone of B16 melanoma cell line was obtained originally from Dr. Jean Starkey (Montana State Univ., Bozeman, MT), and clones were reselected in syngeneic C57/BL mice according to their metastatic potential. C57/BL mice were maintained in plastic cages under filtered air atmosphere and provided with water and food pellets ad lib. Cells were cultured in RPMI 1640 supplemented with 2 mM glutamine and 10% fetal calf serum (FCS), and detached with phosphate buffered saline (PBS) containing 2 mM EDTA. Viability was tested by trypan blue exclusion test.

B. Effects of Oligosaccharide on Metastatic Potential

A suspension of BL6 cells (1-3 x $10^6$ cells/ml RPMI 1640 medium) was prepared and aliquots were incubated in the presence or absence of various oligosaccharides at various concentrations, at 37°C for 5-10 minutes. Following incubation, typically, 3 x $10^4$ or 2 x $10^4$ cells (with or without oligosaccharide pretreatment) per 200 μl were injected via tail vein into 8-week-old female mice. After 18-21 days, mice were killed, lungs were fixed in 10% formaldehyde in PBS (pH 7.4), and tumor cell colonies were counted under a dissecting microscope, thus providing background values of metastatic melanoma colony number in lung under these conditions. Data on number and size of colonies were statistically treated by the analysis of variance (ANOVA) procedure. Colonies with a diameter of 1 mm or greater were considered large-size and those with a diameter less than 1 mm were considered small-size.

For one experiment, BL6 cells were incubated with various concentrations of lactose, lacto-N-tetrose (Galβ1→3GlcNAcβl→3Galβ1→4GLc), methyl β-D-lactoside, or phenyl β-D-thiolactoside for various durations. In the majority of experiments, a concentration of 0.1 M was used and cells were incubated at 37°C for 10 minutes, separated from sugar-containing medium by mild centrifugation at 400 x g for 10 minutes, resuspended in RPMI 1640, and injected (3 x $10^4$ cells in 0.2 ml suspension) via tail vein. For some experiments, 2 x $10^4$ cells were injected and colonies were counted at 21 days. Viability and cell growth ability of BL6 cells after incubation in various sugar solutions were tested by trypan blue exclusion test, and by plating in RPMI 1640 culture under normal conditions *in vitro*, as well as by subcutaneous inoculation in age-matched C57/BL mice in order to test tumor growth.

Lactose and lacto-N-tetrose showed 26% and 36% reductions, respectively, of metastatic colonies in lung when BL6 cells were preincubated with these sugars followed by intravenous injection of cells under identical conditions. Treatment of BL6 cells with 0.1 M, 0.01 M, or 0.005 M methyl β-D-lactoside under the same conditions as above resulted in (respectively) a 43%, 16%, and 8% reduction of metastatic lung colony number compared to control. The significant reduction caused by 0.1 M methyl β-D-lactoside was reproduced in three separate experiments and the reduction was found to be consistently between 35% and 45%.

In a second, completely independent series of experiments, treatment with methyl β-D-lactoside or phenyl β-D-thiolactoside under different conditions also produced a significant reduction of metastatic colonization, i.e., total colony number was reduced to 35% or 50% of control value following preincubation with methyl β-D-lactoside or phenyl β-D-thiolactoside respectively. Reduction of larger-size colonies was more apparent than that of smaller colonies in all experiments, particularly those with phenyl β-D-thiolactoside (Figure 1). Methyl β-D-lactoside and phenyl β-D-thiolactoside both showed a slight *in vitro* stimulatory effect on cell number increase and on thymidine incorporation. Thus, the inhibitory effect on tumor deposition is not related to the effect on cell growth *in vitro* or *in vivo*.

In a separate experiment, the effect of methyl β-D-lactoside on melanoma cell metastasis was determined after administration of the oligosaccharide, followed by inoculation with tumor cells. Specifically, a one ml dosage of methyl β-D-lactoside (at a concentration of 0.25 M or 0.5 M) was injected intraperitoneally in mice. After 10 minutes, B16 melanoma cells were injected intravenously. Lung colonies were counted 19 days later. Injection of methyl β-D-lactoside in advance of inoculation with tumor cells resulted in a significant reduction of lung metastatic colony formation (Figure 2).

In another separate experiment, mouse melanoma B16 variants showing different degrees of metastatic potential (BL6/F10/F1/WA4) showed the same order of expression of GM3 ganglioside, which was previously identified as a melanoma-associated antigen (Hirabayashi et al., J. Biol. Chem. 260:13328, 1985; Nores et al., J. Immunol. 139:3171, 1987) . GM3 interacts with LacCer, which is highly expressed on endothelial cells. The order of adhesion of the B16 variants onto LacCer-coated solid phase or onto endothelial cells was also in the same order as their metastatic potential. In contrast, integrin-dependent adhesion of the B16 variants was approximately equal for BL6, F10, and F1 (see Figure 4). These observations suggest that B16 adhesion of LacCer is based on molecular GM3-LacCer interaction. It has also been demonstrated that B16 melanoma adhesion on endothelial cells is inhibited not only by methyl-β-lactoside but also by LacCer liposome, Gg3Cer liposome, and GM3 liposome (see Figure 5).

In addition, the observations on the metastasis-inhibitory effect of methyl-β-lactoside noted above have been extended to separate methyl-β-lactoside injection, i.e., tumor cells were intravenously injected, followed by intraperitoneal injection of methyl-β-lactoside. In these experiments, injection of 0.25-0.5 M methyl-β-lactoside reduced lung metastatic colony number by 40%-70% (see Figure 6; A=PBS control, B=0.25 M Me-β-lactoside; C=0.5 M Me-β-lactoside; D=0.5 M lactose; E=0.25 M N-acetyllactosamine; F=0.5 M Me-β-galactoside; intraperitoneal injection).

Example 3

EXPRESSION OF SIALOSYL-DIMERIC $LE^x$ ON HUMAN LUNG ADENOCARCINOMA CELL LINES AND METASTATIC POTENTIAL

A. Cell Lines

KUM-LK-2 is a human non-adenocarcinoma cell line characterized as producing spontaneous lung metastasis in nude mice. After screening 35 human carcinoma cell lines grown in nude mice, only this cell line produced metastatic deposits in nude mouse lung. KUM-LK-2 was used as the parent cell line to obtain, by limiting dilution technique, sub-cell lines producing lung metastasis upon IV injection.

The procedure for the limiting dilution technique was as follows. KUM-LK-2 was cultured in RPMI 1640 medium (GIBCO, Grand Island,NY) supplemented with 10% FCS (Hyclone, Logan, UT) at 37°C in a 5% $C0_2$/95% air atmosphere. Cells were treated briefly with 2 mM EDTA solution and washed twice with RPMI 1640 to make a single cell suspension in RPMI with 10% FCS. Cell viability was > 98% as determined by trypan blue exclusion staining. A cell suspension containing 1 cell per 100 μl was transferred to each well of a 96-well microtiter plate (Corning Glass Works, Corning, NY) and cultured continuously for 24 hours. Each well was then examined by phase contrast microscopy.

Three cell lines (HAL-8, HAL-24, and HAL-33) with different metastatic potentional ("MP") were selected out of 25 clones obtained by limiting dilution technique on the basis of stable cell morphology. These 25 clones were originally selected from 63 clones showing stable morphology as well as consistent *in vitro* cell growth. All of these clones produced spontaneous lung metastasis. However, upon I.V. injection, clear differences were observed among the clones in terms of lung metastatic deposit formation. Two clones with high MP, five with low MP, and 18 with no MP were distinguished. Through repeated selection by I.V. injection of these clones, the most stable sub-cell lines showing consistent MP were established. These were HAL-8, -33, and -24, showing high, low, and no MP, respectively, to nu/nu mouse lung (see Table 1 below). Judging by macroscopic and microscopic examination, none of these three sub-cell lines showed metastasis in other organs or lymph nodes. The sub-cell lines represent stable variants originally present in KUM-LK-2. Based on chromosome analysis, these subclones are independent.

Table 1

Metastatic potential of clones HAL-8, -24, and -33 in nude mice.[a]

| Clone | # generations | #lung nodules on day 56[b] |
|---|---|---|
| HAL-8 | 15 | 15.8 (8-23) |
| | 22 | 15.0 (10-22) |
| | 46 | 16.3 (11-25) |
| HAL-24 | 15 | 0 |
| | 22 | 0 |
| | 46 | 0 |
| HAL-33 | 15 | 4.3 (3-7) |
| | 22 | 5.1 (2-8) |
| | 46 | 5.8 (3-8) |

[a] Nude mice were injected (2 x $10^5$ cells) in the tail vein at various generation times as indicated. 56 days after injection, mice were killed and metastatic nodules on lung surface were counted under dissecting microscope.

[b]Mean of 6 animals (range in parentheses)

B. Expression of Cell Surface Carbohydrate Epitopes

The cell surface expression of various carbohydrate epitopes was analyzed by cytofluorometry using various monclonal antibodies (MAbs) directed to $Le^x$ (MAb SH1), sialosyl-$Le^x$ (MAb SH4), sialosyl-dimeric $Le^x$ (MAb FH6), T (MAb HH8), Tn (MAb 1E3), and sialosyl-Tn (MAb TKH2). All antibodies used were culture supernatants from their respective hybridomas, adjusted as 10 μg/ml of immunoglobulin. The structures of sialosyl-$Le^x$ (structure 1), sialosyl-dimeric-$Le^x$ (structure 2), dimeric-$Le^x$ (Structure 3), trifucosyl-$Le^y$ (structure 4), $Le^b$ (structure 5,) H (structure 6), SA-$Le^a$ (structure 7), SA-Tn (structure 8), disialosyl-$Le^a$ (structure 9), monosialosyl-$Le^a$ II (structure 10), GM3 (structure 11), SPG (structure 12), and $Le^a$ (structure 13) are shown below. In these structures, R represents a carrier molecule.

Structure 1:

```
NeuAcα2→3Galβ1→4GlcNAcβ1→3Galβ1→4GlcNAcβ1→3Galβ1→4GLcβ1→R
                      3
                      ↑
                    Fucα1
```

Structure 2:

```
NeuAcα2→3Galβ1→4GlcNAcβ1→3Galβ1→4GlcNAcβ1→3Galβ1→4GLcβ1→R
                      3                    3
                      ↑                    ↑
                    Fucα1                Fucα1
```

Structure 3:

```
Galβ1→4GlcNAcβ1→3Galβ1→4GlcNAcβ1→3Galβ1→4Glcβ1→R
         3                     3
         ↑                     ↑
      Fucα1                 Fucα1
```

Structure 4:

```
Galβ1→4GlcNAcβ1→3Galβ1→4GlcNAcβ1→3Galβ1→4Glcβ1→R
  2      3                 3
  ↑      ↑                 ↑
Fucα1  Fucα1             Fucα1
```

Structure 5:

```
Fucα1→2Galβ1→3GlcNAcβ1→3Galβ1→R
                 4
                 ↑
              Fucα1
```

Structure 6:

Fucα1→2Galβ1→3GlcNAcβ1→3Galβ1→R

Structure 7:

```
NeuAcα2→3Galβ1→3GlcNAcβ1→3Galβ1→R
                   4
                   ↑
                Fucα1
```

Structure 8:

NeuAcα2→6Ga1NAcαl→O-Ser/Thr

Structure 9:

```
                 NeuAcα2
                       ↓
                       6
NeuAcα2→3Galβ1→3GlcNAcβ1→3Galβ1→R
                       4
                       ↑
                    Fucα1
```

Structure 10:

```
        NeuAcα2
              ↓
              6
Galβ1→3GlcNAcβ1→3Galβ1→R
              4
              ↑
          Fucα1
```

Structure 11:

NeuAcα2→3Galβl→4Glcβl→Cer

Structure 12:

NeuAcα2→3Galβ1→4GlcNAcβ1→3Galβ1→R

Structure 13:

```
Galβ1→4GlcNAcβ1→3Galβ1→R
          3
          ↑
      Fucα1
```

Cells were detached from culture flask with 0.25% trypsin, 2mM EDTA solution; 1 x $10^5$ cells were prepared for each MAb treatment. Cells were incubated with a MAb for 1 hour at 4°C and washed 2 times with RPMI 1640. Goat anti-mouse IgG or IgM-FITC (Boehringer-Mannheim, Indianapolis, IN), diluted 50 times with PBS, was then added and incubated 30 minutes at 4°C. Finally, cells were washed 3 times, resuspended with PBS, and applied to an EPICS PROFILE flow cytometry (Epics, Hialeah, FL). These experiments were repeated with three different cell generations.

Patterns of expression of six carbohydrate epitopes (defined by their respective Mabs) on sub-cell lines HAL-8, -24, and -33 showed nearly identical profiles (as did the protein profiles for the three sub-cell lines), except in the case of sialosyl-dimeric-$Le^x$. In particular, HAL-8, -24, and -33 were found to highly and equally express sialosyl-$Le^x$ and sialosyl-Tn structures. Each of the three lines expressed low quantities of $Le^x$ and Tn, and did not express T. In contrast, expression of sialosyl-dimeric $Le^x$ was high on HAL-8, moderate on HAL-33, and low on HAL-24.

C. Inhibition of Metastasis by Sialidase Treatment of Cells

Cells were detached using 2 mM EDTA in PBS, washed, and resuspended in 9 volumes of PBS. One ml of cell suspension was incubated 5 minutes at 37°C with 0.2 U/ml of *Clostridium perfringens* sialidase (type X, Sigma Chemical Co., St. Louis, MO). After incubation, cells were washed three times, resuspended with RPMI 1640, and investigated for MP and expression of sialosyl-dimeric-$Le^x$. MP of HAL-8 and -33 was completely inhibited by sialidase treatment of cells (see Table 2 below). Expression of sialosyl-dimeric-$Le^x$ appears to play an important role in blood-borne metastasis.

Table 2

Effect of sialidase treatment on metastatic potential of clones HAL-8 and -33.[a]

| Treatment | Clone | # lung nodules on day 56[b] |
|---|---|---|
| Control (PBS) | HAL-8 | 16.3 (9-24) |
| | HAL-33 | 4.6 (3-7) |
| Sialidase | HAL-8 | 0 |
| | HAL-33 | 0 |

[a] Nude mice were injected (2 x $10^5$ cells) in the tail vein. 56 days after injection, mice were killed and metastatic nodules on lung surface were counted under dissecting microscope.

[b] Mean of 6 animals (range in parentheses).

EXAMPLE 4

IDENTIFICATION OF CARBOHYDRATE EPITOPES CAPABLE OF BINDING TO THE LECTIN DOMAIN OF GMP-140

A. Preparation of Platelets

Platelets were isolated from"platelet-rich plasma"obtained from the Oregon Red Cross (Portland, OR), and contaminating red blood cells were removed by centrifugation at 80xg for 10 min. Platelets were centrifuged at 300xg for 10 min, and suspended in Tyrode's buffer (pH 6.5) containing 22 mM citrate buffer with 0.35% bovine serum albumin (BSA). Platelet suspension ($1x10^8$/ml) was incubated (pH 7.2, 37°C, 5 min) after addition of thrombin (final concentration 1 U/ml). The mixture was then incubated at 37°C for 10 min without stirring. Thrombin-activated platelets were fixed with an equal volume of 2% formaldehyde in phosphate-buffered saline (PBS), pH 7.2, and washed 2x with PBS containing 1% BSA. Activated platelets (but not non-activated platelets) showed strong reactivity with 2.5 µg/ml anti-GMP-140 MAb AC1.2 (isotype $IgG_1$; Beckton-Dickinson, San Jose, CA) when incubated at 37°C for 30 min, followed by reaction with 50 µl of fluorescent-labeled goat anti-mouse Ig (Tago, Burlingame, CA). Flow cytometric profiles of activated vs. non-activated platelets with Mab AC1.2 are shown in Figs. 8A-8D.

Activated and non-activated platelets were fixed with paraformaldehyde in $Ca^{2+}$-free PBS, pH 7.2, washed 2x with $Ca^{2a+}$-containing PBS with 1% BSA, resuspended in $CA^{2a+}$-PBS with 1% BSA and 0.1% azide, and the number of platelets adjusted to ≈$1x10^9$/ml. The cell suspension was stored at 4°C and the binding assay performed within 24 hr.

B. Preparation of Fluorescent Polystyrene Beads Coated with GSLs

Fluorescent polystyrene latex beads were obtained from Molecular Probe, Inc., Eugene, OR. They were yellow-green fluorescent beads with a sulfate group at the surface, diameter ≈0.5 µm (actually 0.486 µm). $1x10^9$ beads in 30 µl ETOH were added to 10 µg of GSL solution in 200 µl C=M, mixed well, and dried under an $N_2$ stream. The residue was resuspended in 200 µl ethanol, sonicated briefly, and dried under an $N_2$ stream. The dried residue was suspended in 2 ml $Ca^{2+}$-PBS with 3% BSA and 0.1% azide, sonicated for 10 min, and allowed to stand at 37°C for 60 min to block the surface with BSA. The suspension was centrifuged at 3000xg for 10 min, the bead pellet was washed 2x with $Ca^{2+}$-PBS with 1% BSA and azide, and finally suspended in 500 µl of this medium and stored at 4°C.

C. Binding Assay

20 µl of platelet (non-activated or activated) suspension ... paraformaldehyde-fixed -- containing ≈$2x10^7$ platelets was mixed with 10 µl of fluorescent GSL-coated beads -- containing ≈$2x10^7$ beads, mixed well, 20 µl of fixed platelet suspension (≈ $2x10^7$ cells) was mixed with 10 µl of fluorescent GSL-coated beads (≈ $2x10^7$ beads) and allowed to stand at 37°C for 30 min. The platelet suspension was mixed with 200 µl $Ca^{2+}$-PBS and analyzed by flow cytometry (EPICS Profile, Coulter Cytometry, Hialeath, FL).

D. Data Analysis from Flow Cytometric Profile

Flow cytometric analyses of platelets alone and beads alone were performed for a gating set to include most signals produced by platelets by excluding signals produced by free beads. The binding index (BI) was calculated as mean fluorescence intensity (MFI) of platelets incubated with fluorescent GSL-coated beads, divided by MFI of platelets incubated with fluorescent non-GSL-coated (control) beads. BI values for various GSLs are shown in Table 3 and Figure 9. In Figure 9, the hatched bars represent non-activated platelets and the open bars represent activated platelets. The ratio of the binding index (BI) of activated/non-activated platelets for SA-$Le^x$, SA-$Le^a$, SPG., GM3 and $Le^x$ is also shown under "Ratio A/NA" column.

Table 3

Binding index of thrombin-activated platelets to GSL-coated, sulfate-containing polystyrene a beads

| GSL | Activated platelets | Ratio (Activated/ activated) | Non- |
|---|---|---|---|
| GM3 | 1.0 ± 0.1 | 0.7 ± 0.5 | |
| SA-$Le^x$ | 4.2 ± 1.0 | 4.2 ± 0.5 | |
| SA-$Le^a$ | 8.1 ± 1.0 | 6.1 ± 0.2 | |
| SPG | 0.8 ± 0.2 | 0.7 ± 0.2 | |
| $Le^x$ | 1.1 ± 0.3 | 1.0 ± 0.3 | |

[a] Values represent means of four separate experiments.

E. Inhibition of Platelet Binding to Fluorescent GSL-Coated Beads by MAbs

1. Anti-GMP-140. Platelets were incubated with anti-GMP-140 MAb IOP62 (Immunotech, Marseille, France) at 37°C for 30 min, and a binding assay was performed using GSL-coated beads as described above. Non-specific mouse IgG (10 µg/ml) was used in a control binding assay.

2. Anti-SA-$Le^a$ and anti-SA-$Le^x$ MAbs. 10 µl of SA-$Le^a$-coated beads ($2x10^7$) were incubated with 20 µl of anti-SA-$Le^a$ MAb CA19-9 (20 µg/ml) (mouse $IgG_1$; Signet Laboratories, Dedham, MA) at room temperature for 60 min and used for the platelet binding assay. Anti-SA-$Le^x$ MAb SNH4 and non-specific mouse IgG were used as controls.

The results are shown in Figure 10, where the abscissa represents percent inhibition and column 1 represents anti-GMP-140 140 MAb IOP62, column 2 represents anti-SA-$Le^a$ MAb CA19-9, column 3 represents anti-SA-$Le^x$ MAb SNH4, and column 4 represents normal mouse IgG.

F. Results

Activated platelets showed high expression of GMP-140 as evidenced by high reactivity with anti-CD62

MAb (Figs. 8A-8D). Activated platelets expressing GMP-140 showed strong binding with fluorescent beads coated with SA-$Le^x$ (Fig. 9, Table 3). Binding of platelets to beads coated with SA-$Le^x$ was observed, but to a much lower degree than with SA-$Le^a$ (Fig. 9, Table 3). No binding was observed to beads coated with other GSLs. Further, the binding of platelets to SA-$Le^a$ coated beads was inhibited by anti-GMP-140 MAb and anti-SA-$Le^a$ MAb, but not by anti-SA-$Le^x$ MAb (Fig. 10).

EXAMPLE 5

EFFECT OF VARIOUS MONOCLONAL ANTIBODIES ON ADHESION OF HUMAN COLON CARCINOMA COLO205 CELLS TO INTERLEUKIN-1-ACTIVATED HUMAN UMBILICAL VEIN ENDOTHELIAL CELLS IN A DYNAMIC FLOW SYSTEM

Adhesion was measured using the dynamic flow experimental system shown in Figures 11A to 11D. The number of cells bound during 3 minutes at different shear stresses from 0.4 to 4.8 dynes/$cm^3$ were counted from several fields recorded on videotape. The coefficient of viscosity was 1.0 P, the half channel height was 5.7 X $10^{-3}$ cm, and the channel width was 1.3 cm.

Using this system, various human tumors and monoclonal antibodies directed to various tumor-associated carbohydrate antigens were studied. The results of one study, adhesion of human colon carcinoma Colo205 cells to activated human endothelial cells, is shown in Fig. 12, where the abscissa represents wall shear stress (dynes/$cm^2$) and the ordinate represents cell adhesion (x$10^{-2}$/field).

In Fig. 12, the symbols are as follows: open circles, mixture of irrelevant mouse IgG plus IgM (control); solid triangles, monoclonal antibody CA19-9 directed to monosialosyl-$Le^a$ I; open triangles, monoclonal antibody SNH4 directed to sialosyl-$Le^x$; solid circles, monoclonal antibody FH7 directed to monosialosyl-$Le^a$ II and disialosyl-$Le^a$; and solid squares, mixture of irrelevant mouse IgG plus IgM and non-activated endothelial cells.

The results show that adhesion of Colo205 cells to activated endothelial cells was most strongly inhibited by antibody FH7, particularly at high wall shear stress (5-10 dynes/$cm^2$). In contrast, antibody CA19-9 had no inhibitory effect. These findings suggest that tumor cell adhesion to endothelial cells may proceed via interaction between monosialosyl-$Le^a$ II or disialosyl-$Le^a$ and interleukin-1-activated selectin.

From the foregoing, it will be evident that, although specific embodiments of the invention have been described herein for purposes of illustration, various modifications may be made without deviating from the spirit and scope of the invention. The following numbered paragraphs set out features of at least some embodiments of the invention.

1. A method for inhibiting tumor cell metastasis potential within a biological preparation, comprising:
incubating the biological preparation with at least one agent selected from the group consisting of mimetics of monosialosyl-$Le^a$ I, monosialosyl-$Le^a$ II, disialosyl-$Le^a$, and sialosyl $Le^x$, said agent inhibiting the metastasis potential of the preparation.
2. The method of Paragraph 1, wherein said agent is a mimetic of monosialosyl-$Le^a$ I.
3. The method of Paragraph 1, wherein said agent is a mimetic of monosialosyl-$Le^a$ II.
4. The method of Paragraph 1, wherein said agent is a mimetic of disialosyl-$Le^a$.
5. The method of Paragraph 1, wherein said agent is a mimetic of sialosyl $Le^x$.
6. The method of any one of Paragraphs 2 to 5, wherein the agent coupled to poly(ethylene glycol).
7. A method for inhibiting tumor cell metastasis potential in a warm-blooded animal, comprising:
administering to a warm-blooded animal an effective amount of at least one agent selected from the group consisting of mimetics of monosialosyl-$Le^a$ I, monosialosyl-$Le^a$ II, disialosyl-$Le^y$, and sialosyl $Le^x$, said agent inhibiting the metastasis potential in the warm blooded animal.
8. The method of Paragraph 7, wherein said agent is a mimetic of monosialosyl-$Le^a$ I.
9. The method of Paragraph 7, wherein said agent is a mimetic of monosialosyl-$Le^a$ II.
10. The method of Paragraph 7, wherein said agent is a mimetic of disialosyl-$Le^a$.
11. The method of Paragraph 7, wherein said agent is a mimetic of sialosyl $Le^x$.
12. The method of any one of Paragraph 8 to 11, wherein the agent is coupled to poly(ethylene glycol).
13. A conjugate comprising a mimetic selected from the group consisting of mimetics of monosialosyl-$Le^a$ I, mimetics of monosialosyl-$Le^a$ II, mimetics of disialosyl-$Le^y$, and mimetics of sialosyl $Le^x$ coupled to poly(ethylene glycol).
14. The conjugate of Paragraph 13, wherein said mimetic is a mimetic of monosialosyl-$Le^a$ I.
15. The conjugate of Paragraph 13, wherein said mimetic is a mimetic of monosialosyl-$Le^a$ II.
16. The conjugate of Paragraph 13, wherein said mimetic is a mimetic of disialosyl-$Le^y$.
17. The conjugate of Paragraph 13, wherein said mimetic is a mimetic of sialosyl $Le^x$.

18. A method for inhibiting GMP-140-mediated or ELAM-1-mediated cell aggregation or adhesion within a biological preparation, comprising:
incubating the biological preparation with at least one agent selected from the group consisting of mimetics of monosialosyl-$Le^a$ I, monosialosyl-$Le^a$ II, disialosyl-$Le^a$, and sialosyl $Le^x$, said agent inhibiting the cell aggregation or adhesion.
19. The method of Paragraph 18, wherein the cell aggregation or adhesion is due to: (a) adhesion of cells to platelets, (b) adhesion of cells to cells via platelets, (c) adhesion of cells to endothelial cells via platelets, or (d) adhesion of cells to endothelial cells directly via GMP-140 or ELAM-1.
20. The method of Paragraph 19, wherein the cell aggregation or adhesion is GMP-140-mediated.
21. The method of Paragraph 19, wherein the cell aggregation adhesion is ELAM-1-mediated.
22. The method of Paragraph 18, wherein said agent is a mimetic of monosialosyl-$Le^a$ I.
23. The method of Paragraph 18, wherein said agent is a mimetic of monosialosyl-$Le^a$ II.
24. The method of Paragraph 18, wherein said agent is a mimetic of disialosyl-$Le^a$.
25. The method of Paragraph 18, wherein said agent is a mimetic of sialosyl $Le^x$.
26. The method of any of Paragraphs 22 to 25, wherein the agent coupled to poly(ethylene glycol).
27. A method for inhibiting GMP-140-mediated or ELAM-1-mediated cell aggregation or adhesion at a tumor cell site thereby reducing metastatic potential at the site in a warm-blooded animal, comprising
administering to warm-blooded animal an effective amount of at least one agent selected from the group consisting of mimetics of monosialosyl-$Le^a$ I, monosialosyl-$Le^a$ II, disialosyl-$Le^a$ II, disialosyl-$Le^a$, and sialosyl $Le^x$, said agent reducing the metastatic potential at the tumor cell site in said warm-blooded animal.
28. The method of Paragraph 27, wherein the cell aggregation or adhesion is due to: (a) adhesion of tumor cells to platelets, (b) adhesion of tumor cells to tumor cells via platelets, (c) adhesion of tumor cells to endothelial cells via platelets, and (d) adhesion of tumor cells to endothelial cells directly via GMP-140 or ELAM-1.
29. The method of Paragraph 28, wherein the cell aggregation or adhesion is GMP-140-mediated.
30. The method of Paragraph 28, wherein the cell aggregation or adhesion is ELAM-1-mediated.
31. The method of Paragraph 27, wherein said agent is a mimetic of monosialosyl-$Le^a$ I.
32. The method of Paragraph 27, wherein said agent is a mimetic of monosialosyl-$Le^a$ II.
33. The method of Paragraph 27, wherein said agent is a mimetic of disialosyl-$Le^a$.
34. The method of Paragraph 27, wherein said agent is a mimetic of sialosyl $Le^x$.
35. The method of any one of Paragraphs 31 to 34, wherein the agent is coupled to poly(ethylene glycol).
36. A method of inhibiting GMP-140-mediated cell aggregation or adhesion at an inflammation site thereby reducing inflammatory potential at the site in a warm-blooded animal, comprising:
administering to a warm-blooded animal an effective amount of at least one agent selected from the group consisting of mimetics of monosialosyl-$Le^a$ I, monosialosyl-$Le^a$ II, disialosyl-$Le^a$, or sialosyl $Le^x$, said agent reducing the inflammatory potential at the inflammatory site in said warm-blooded animal.
37. The method of Paragraph 36, wherein the cell aggregation or adhesion is due to: (a) adhesion of cells to platelets, (b) adhesion of cells to cells via platelets, (c) adhesion of cells to endothelial cells via platelets, and (d) adhesion of cells to endothelial cells directly via GMP-140.
38. The method of Paragraph 36, wherein said agent is a mimetic of monosialosyl-$Le^a$ I.
39. The method of Paragraph 36, wherein said agent is a mimetic or monosialosyl-$Le^a$ II.
40. The method of Paragraph 36, wherein said agent is a mimetic of disialosyl-$Le^a$.
41. The method of Paragraph 36, wherein said agent is a mimetic of sialosyl $Le^x$.
42. The method of any one of Paragraphs 38 to 41, wherein the agent is coupled to poly(ethylene glycol).

## Claims

1. A method for inhibiting tumor cell metastasis potential within a biological preparation, comprising:
incubating the biological preparation with at least one agent selected from the group consisting of mimetics of monosialosyl-$Le^a$ I, monosialosyl-$Le^a$ II, disialosyl-$Le^a$, and sialosyl $Le^x$, said agent inhibiting the metastasis potential of the preparation.

2. The method of Claim 1 wherein the agent is coupled to poly(ethylene glycol).

3. At least one agent selected from the group consisting of mimetics of monosialosyl-$Le^a$ I, monosialosyl-$Le^a$ II, disialosyl-$Le^y$ and sialosyl-$Le^x$ for use as a medicament.

**4.** At least one agent as claimed in claim 3 for use as a medicament for inhibiting the metastasis potential of a warm blooded animal or inhibiting GMP-140 mediated or ELAM-1 mediated cell aggregation or adhesion at a tumor cell site thereby reducing metastatic potential at the site in a warm blooded animal.

**5.** At least one agent as claimed in claim 3 or 4 coupled to poly(ethylene glycol).

**6.** The use of at least one agent selected from the group consisting of mimetics of monosialosyl-$Le^a$ I, monsialosyl-$Le^a$ II, disialosyl-$Le^y$ and sialosyl $Le^x$ in the manufacture of a medicament for inhibiting the metastasis potential of a warm blooded animal or inhibiting GMP-140 mediated cell aggregation or adhesion at an inflammation site thereby reducing inflammatory potential at the site in a warm blooded animal.

**7.** A use as claimed in claim 6 wherein a, the, or each agent or agents is or are coupled to poly(ethylene glycol).

**8.** A conjugate comprising a mimetic selected from the group consisting of mimetics of monosialosyl-$Le^a$ I, mimetics of monosialosyl-$Le^a$ II, mimetics of disialosyl-$Le^y$, and mimetics of sialosyl $Le^x$ coupled to poly(ethylene glycol).

**9.** A method for inhibiting GMP-140-mediated or ELAM-1-mediated cell aggregation or adhesion within a biological preparation, comprising:
incubating the biological preparation with at least one agent selected from the group consisting of mimetics of monosialosyl-$Le^a$ I, monosialosyl-$Le^a$ II, disialosyl-$Le^a$, and sialosyl Le, said agent inhibiting the cell aggregation or adhesion.

**10.** The method of claim 9, wherein the cell aggregation or adhesion is due to: (a) adhesion of cells to platelets, (b) adhesion of cells to cells via platelets, (c) adhesion of cells to endothelial cells via platelets, or (d) adhesion of cells to endothelial cells directly via GMP-140 or ELAM-1.

**11.** The method of claim 9 wherein the agent is coupled to poly(ethylene glycol).

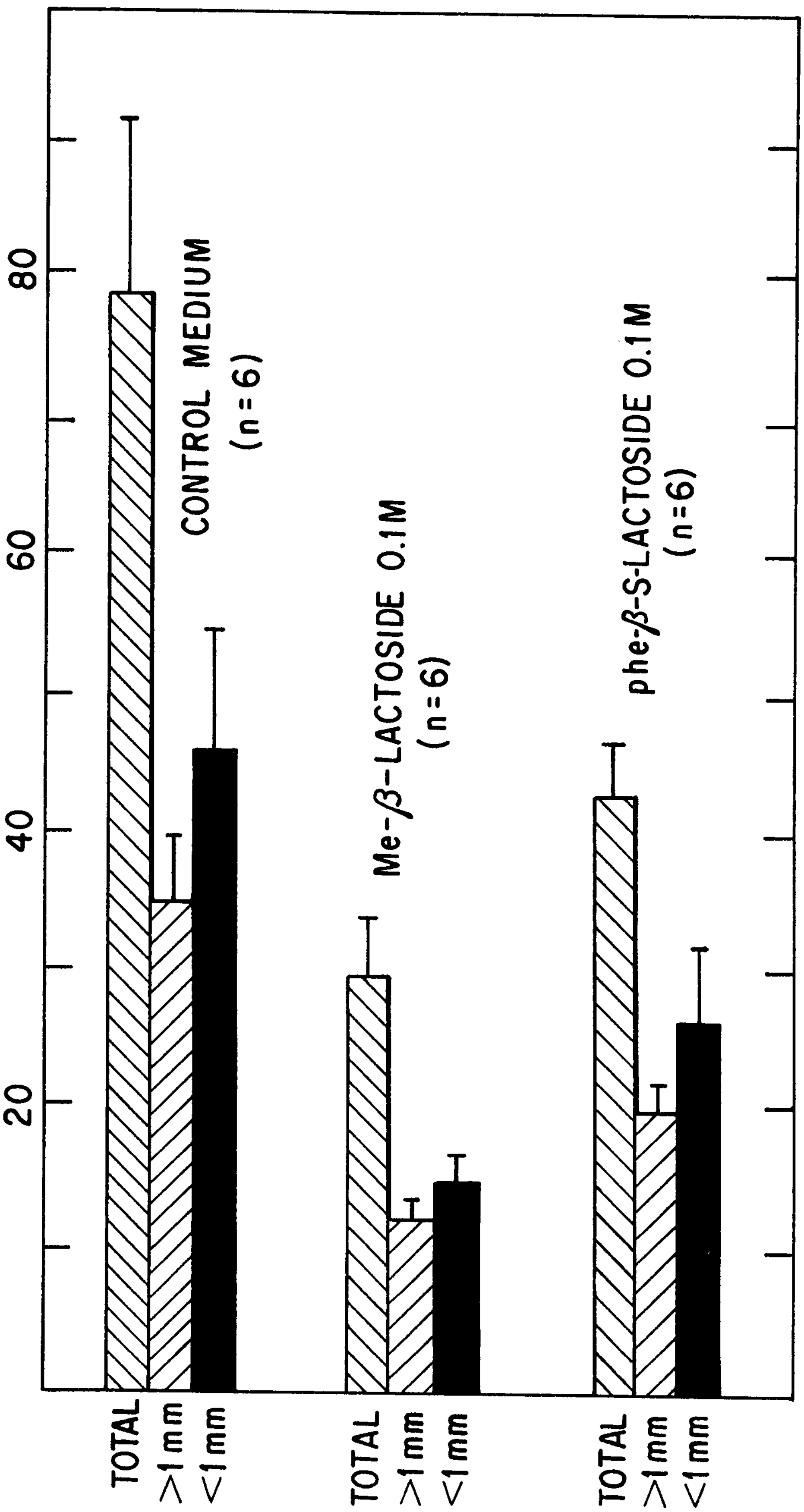
CONTROL MEDIUM
(n=6)
Me-β-LACTOSIDE 0.1M
(n=6)
phe-β-S-LACTOSIDE 0.1M
(n=6)
20
40
60
80
TOTAL
>1mm
<1mm

FIG. 1

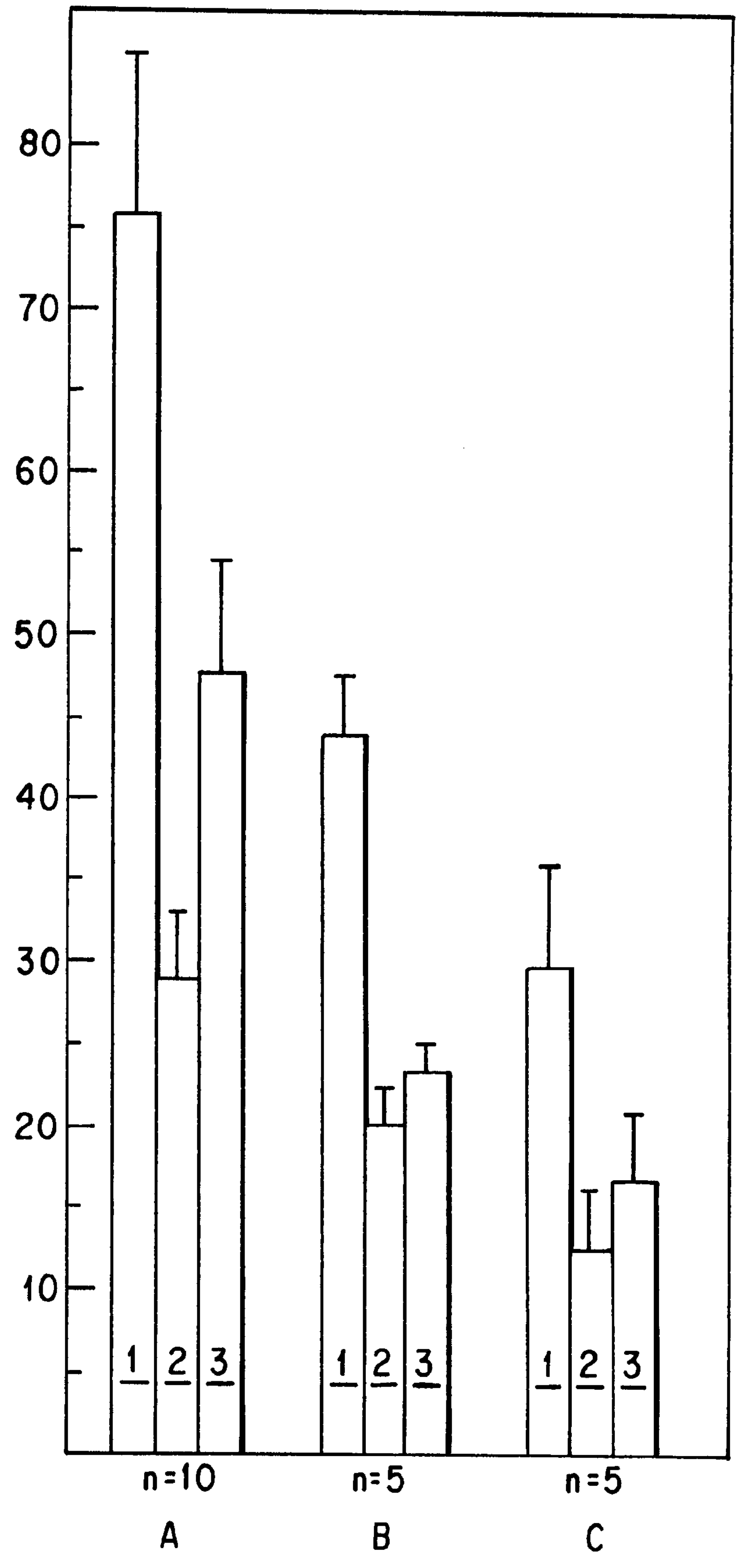


FIG. 2

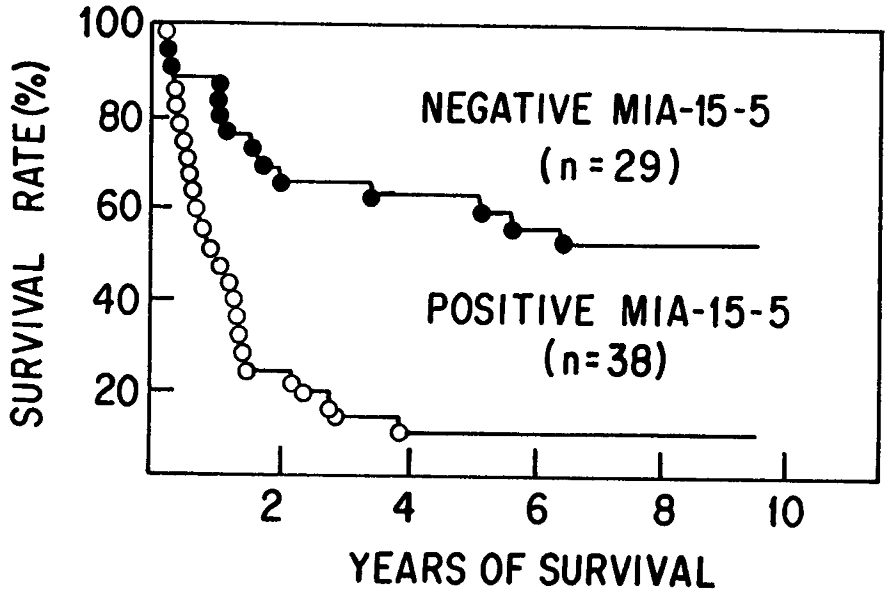
100
80
60
40
20
SURVIVAL RATE (%)
NEGATIVE MIA-15-5
(n=29)
POSITIVE MIA-15-5
(n=38)
2
4
6
8
10
YEARS OF SURVIVAL

FIG. 3A

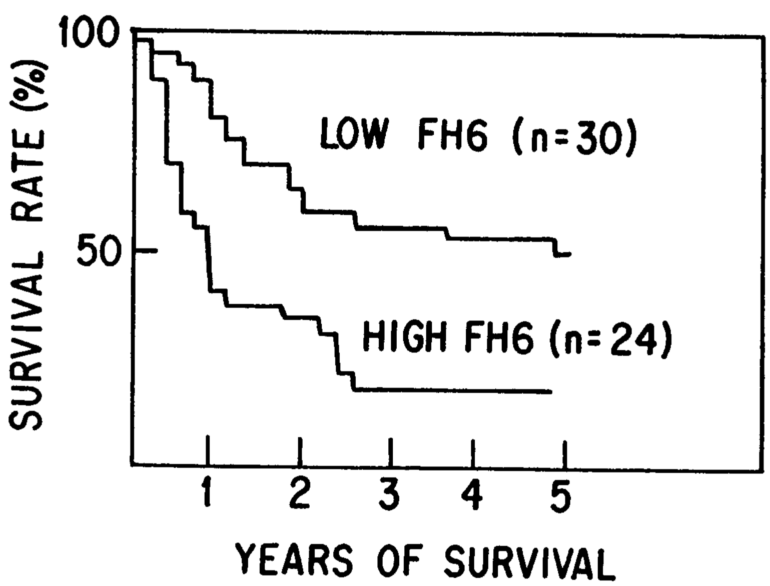
100
50
SURVIVAL RATE (%)
LOW FH6 (n=30)
HIGH FH6 (n=24)
1
2
3
4
5
YEARS OF SURVIVAL

FIG. 3B

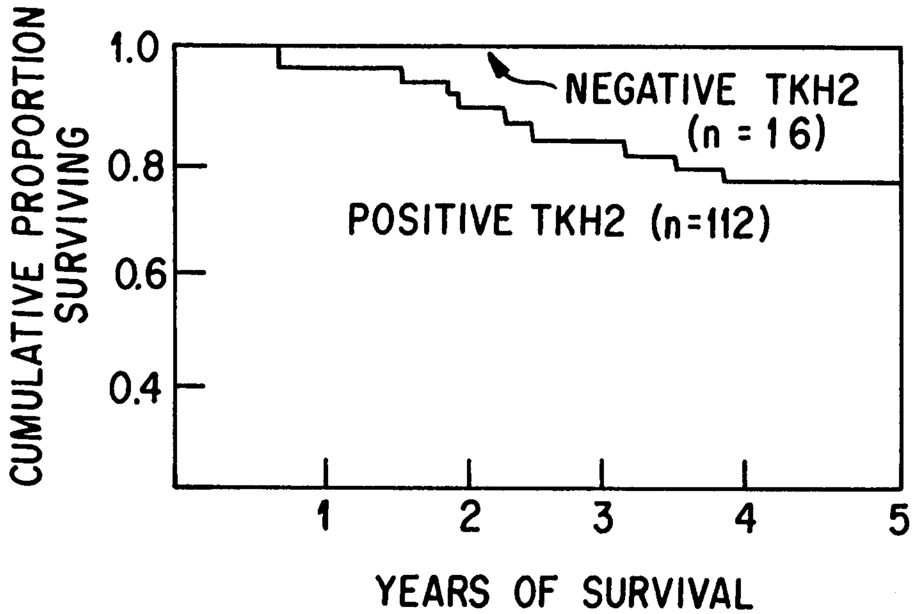
CUMULATIVE PROPORTION SURVIVING
1.0
0.8
0.6
0.4
NEGATIVE TKH2 (n = 16)
POSITIVE TKH2 (n=112)
1
2
3
4
5
YEARS OF SURVIVAL

FIG.3C

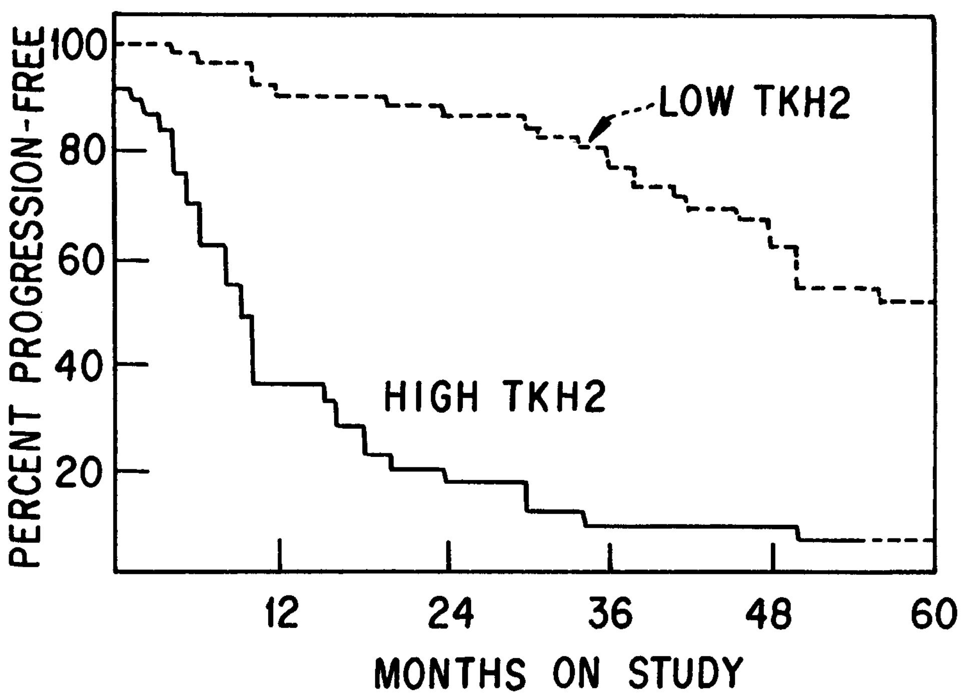
PERCENT PROGRESSION-FREE
100
80
60
40
20
LOW TKH2
HIGH TKH2
12
24
36
48
60
MONTHS ON STUDY

FIG. 3D

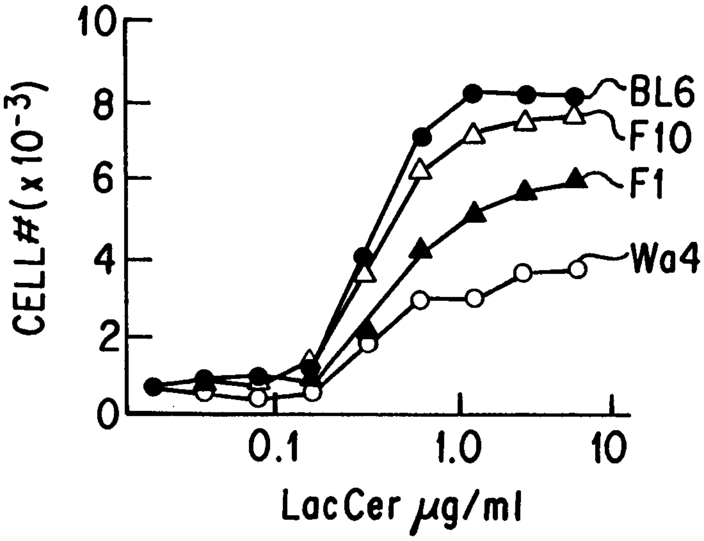


FIG. 4A

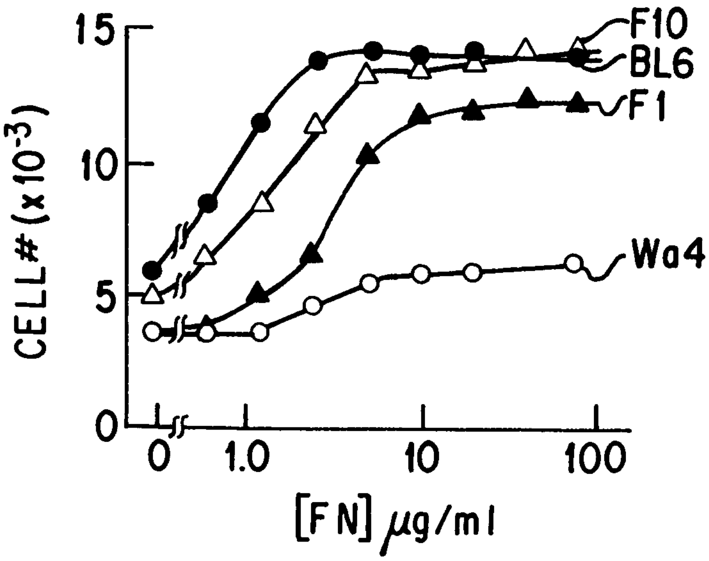


FIG. 4B

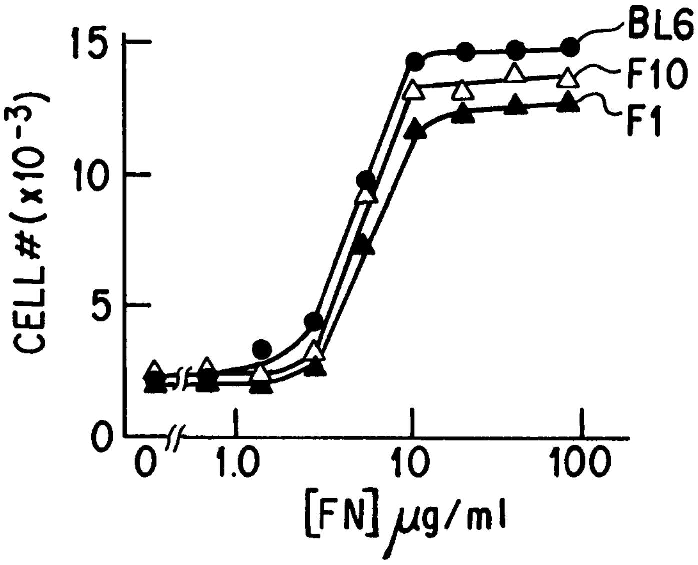


FIG. 4C

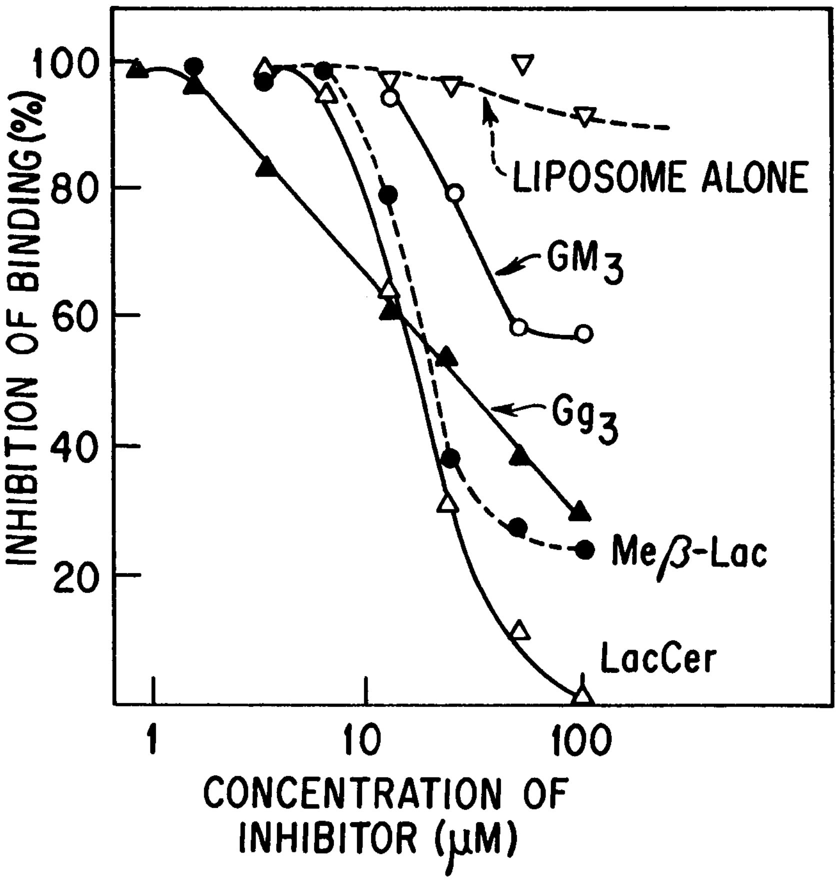
100
80
60
40
20
INHIBITION OF BINDING (%)
LIPOSOME ALONE
GM3
Gg3
Meβ-Lac
LacCer
1
10
100
CONCENTRATION OF
INHIBITOR (μM)

FIG. 5A

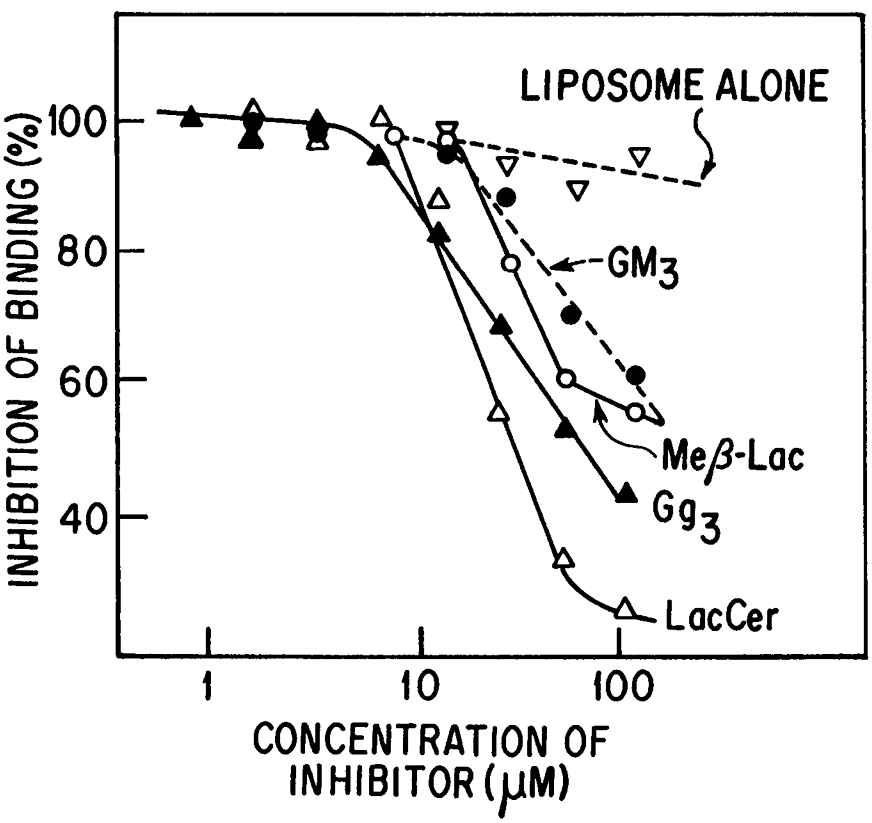
LIPOSOME ALONE
100
80
60
40
INHIBITION OF BINDING (%)
GM3
Meβ-Lac
Gg3
LacCer
1
10
100
CONCENTRATION OF
INHIBITOR (μM)

FIG. 5B

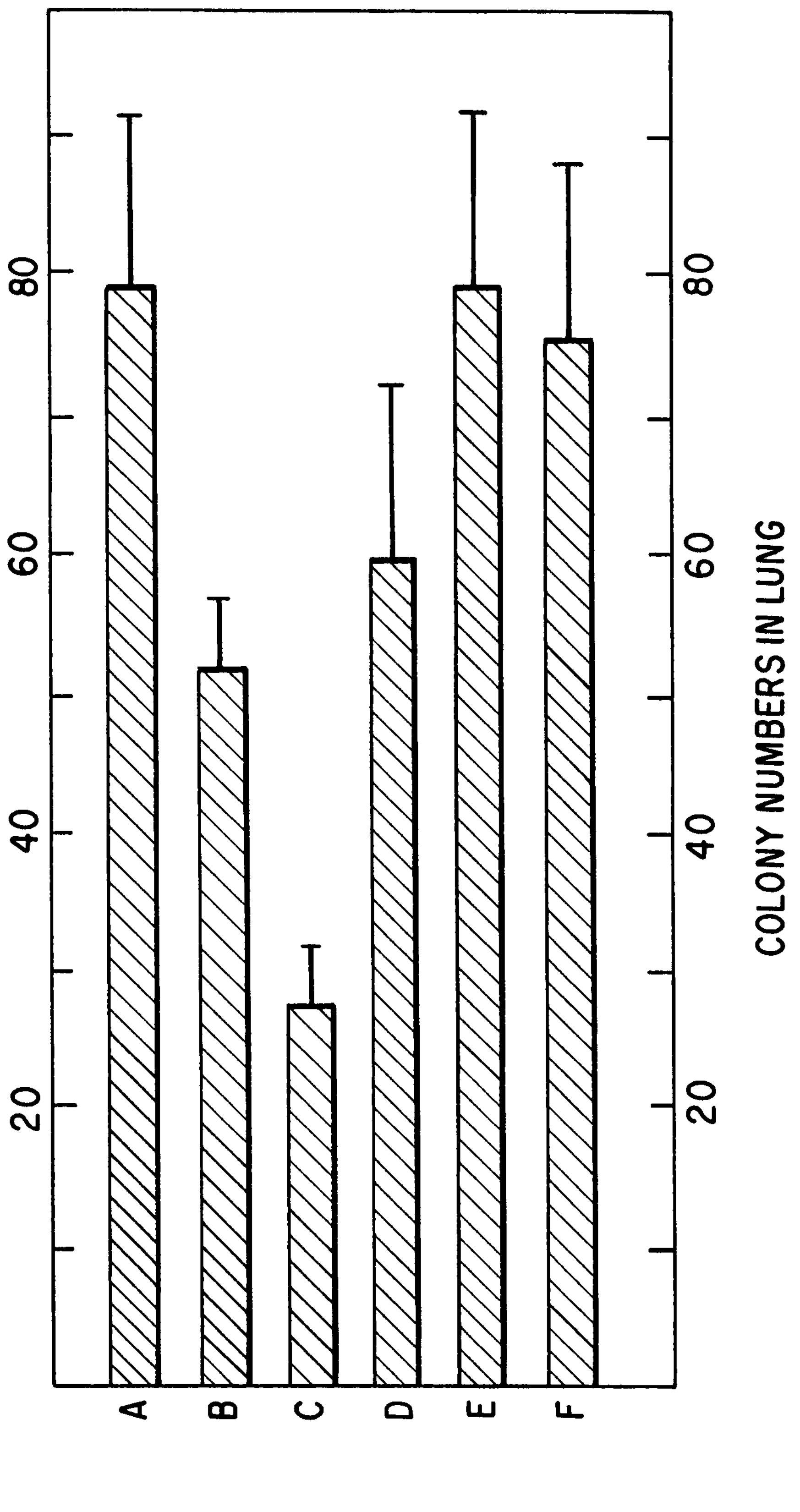


FIG. 6

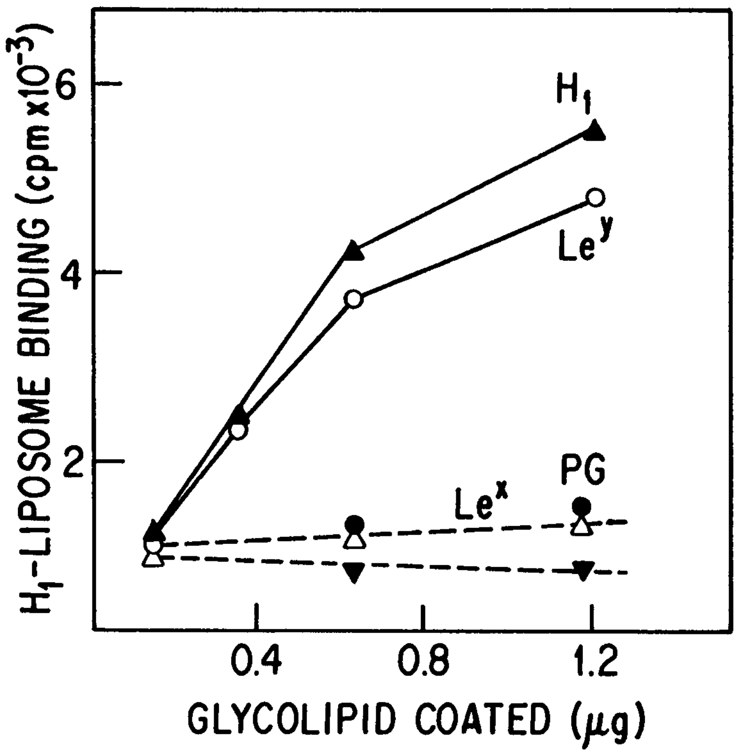

H1-LIPOSOME BINDING (cpm x10-3)
6
4
2
H1
Ley
PG
Lex
0.4
0.8
1.2
GLYCOLIPID COATED (μg)


FIG. 7A

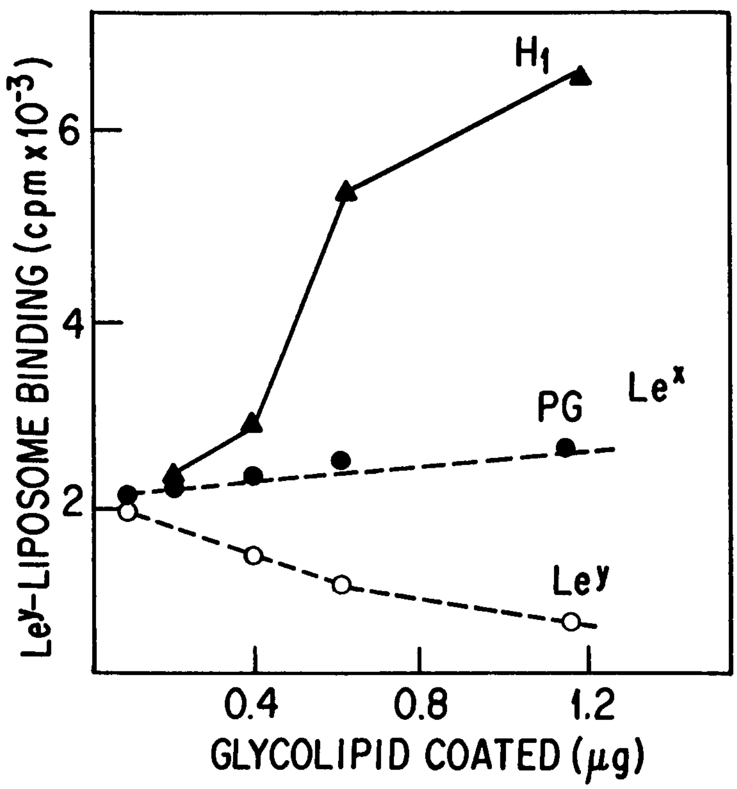

Ley-LIPOSOME BINDING (cpm x 10-3)
6
4
2
H1
PG
Lex
Ley
0.4
0.8
1.2
GLYCOLIPID COATED (μg)


FIG. 7B

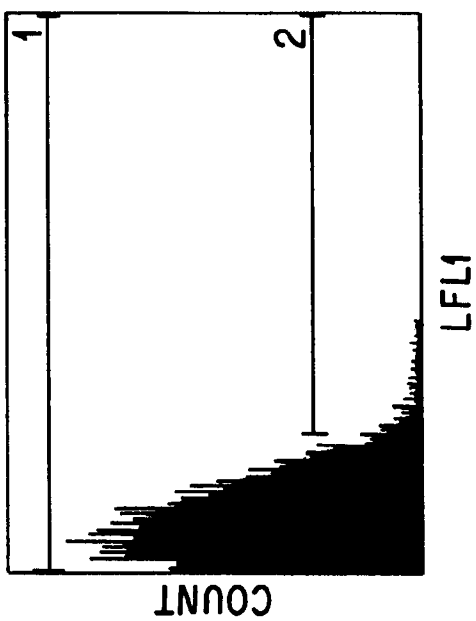


FIG. 8A

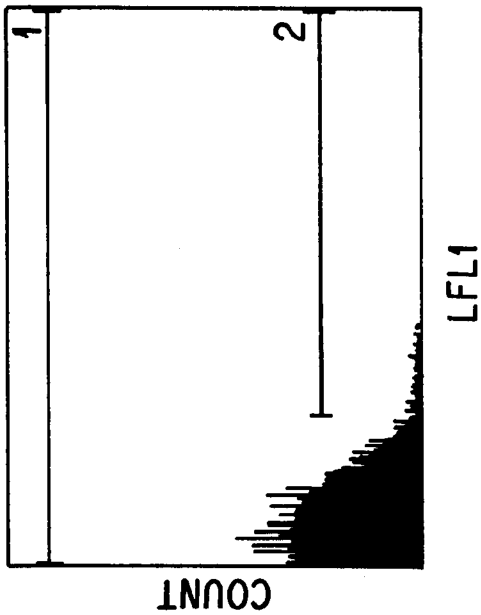


FIG. 8B

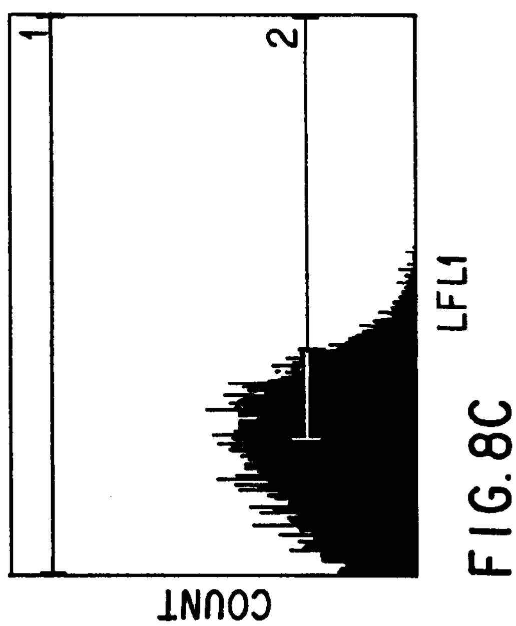


FIG. 8C

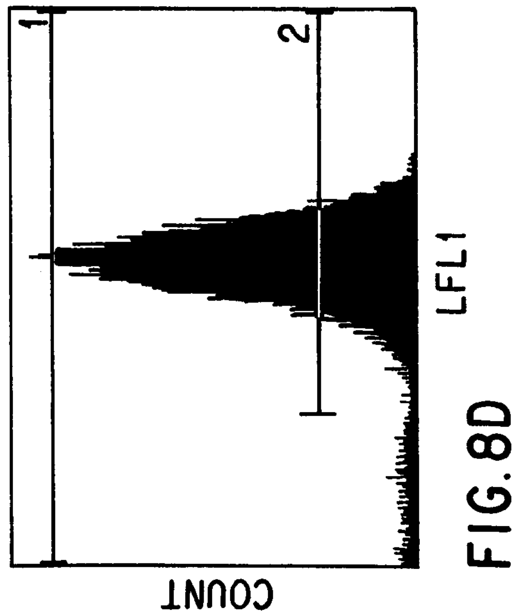


FIG. 8D

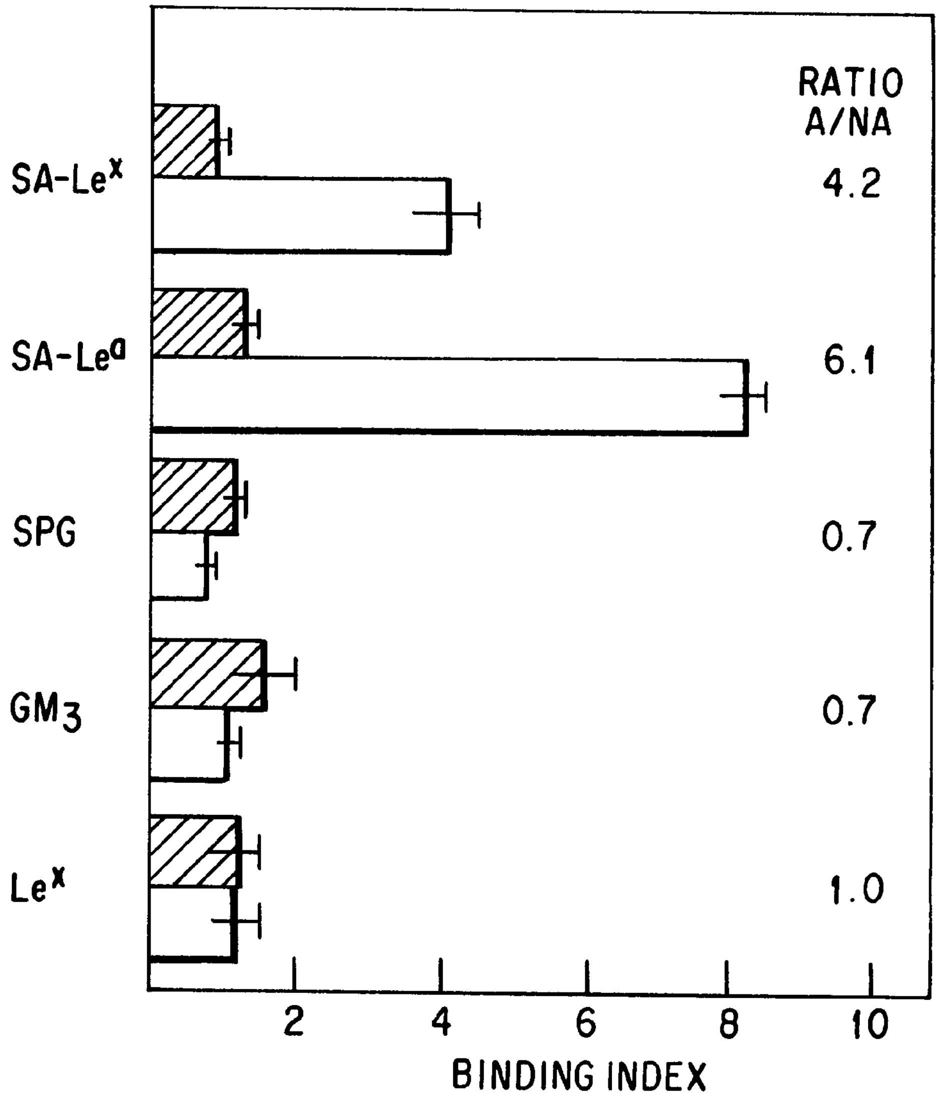
RATIO
A/NA
SA-Le^x
4.2
SA-Le^a
6.1
SPG
0.7
GM3
0.7
Le^x
1.0
2
4
6
8
10
BINDING INDEX

FIG.9

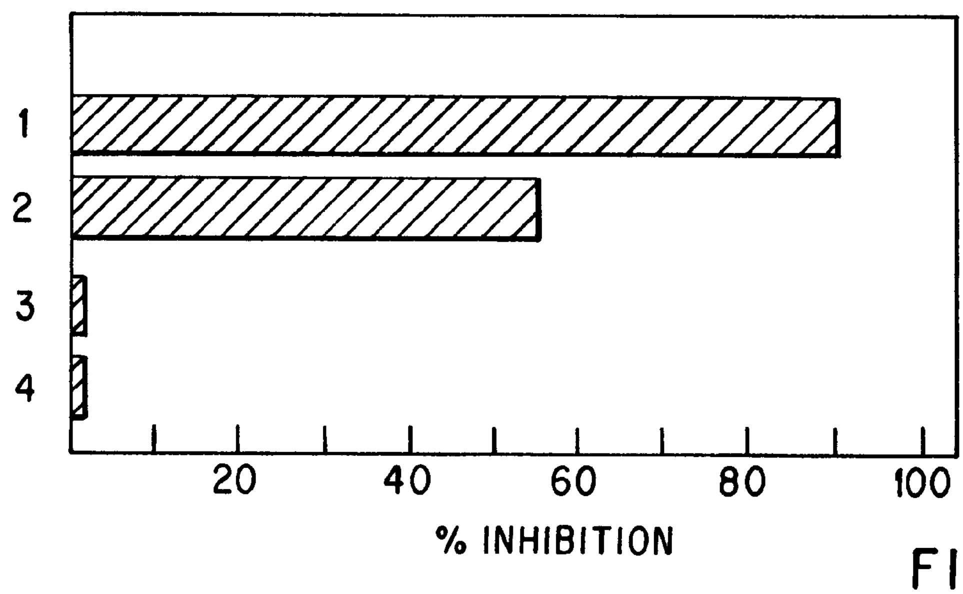
1
2
3
4
20
40
60
80
100
% INHIBITION

FIG.10

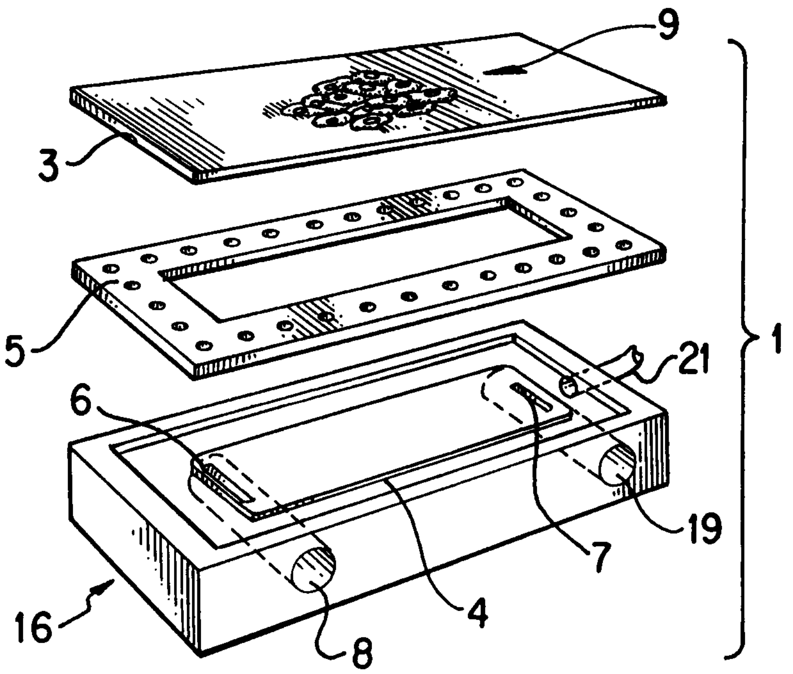


FIG. 11A

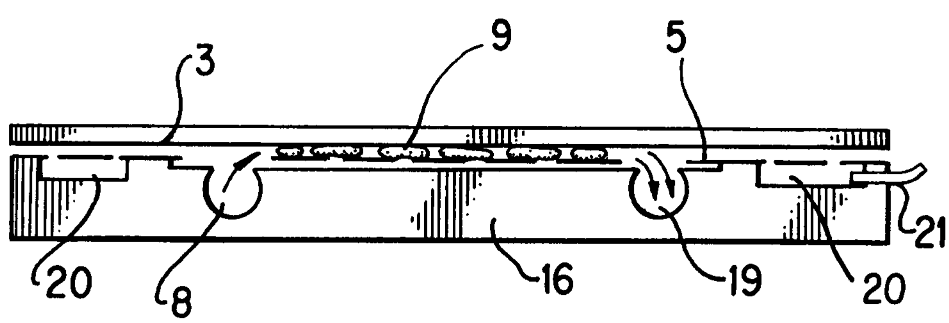


FIG. 11B

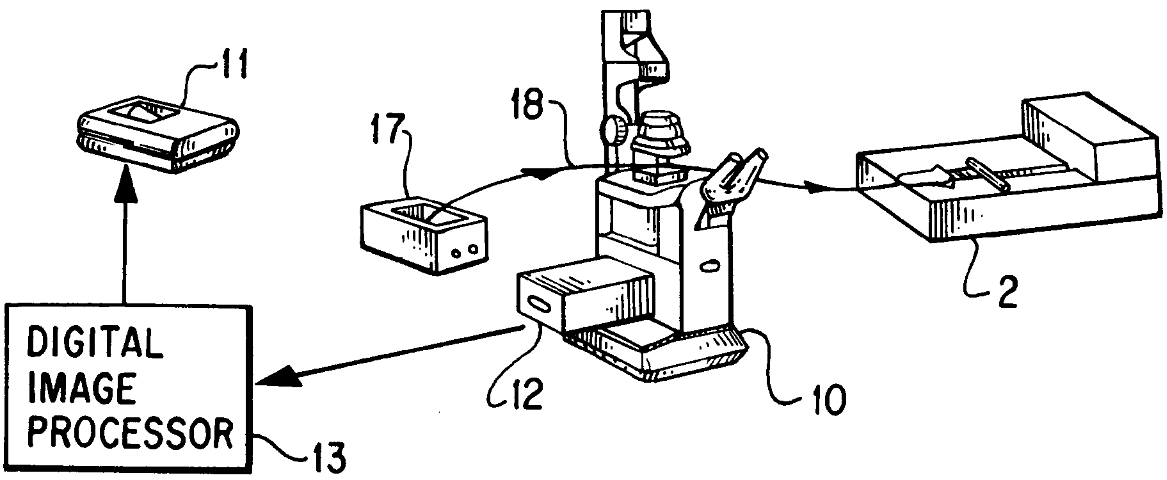


FIG. 11C

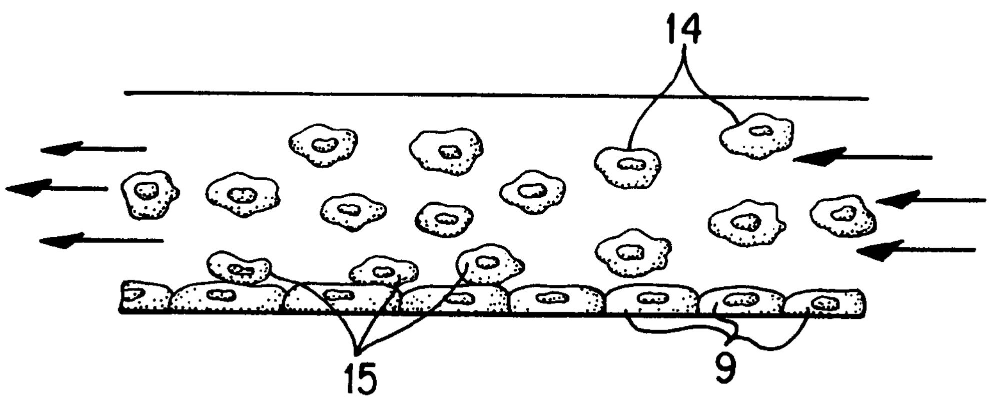


FIG. 11D

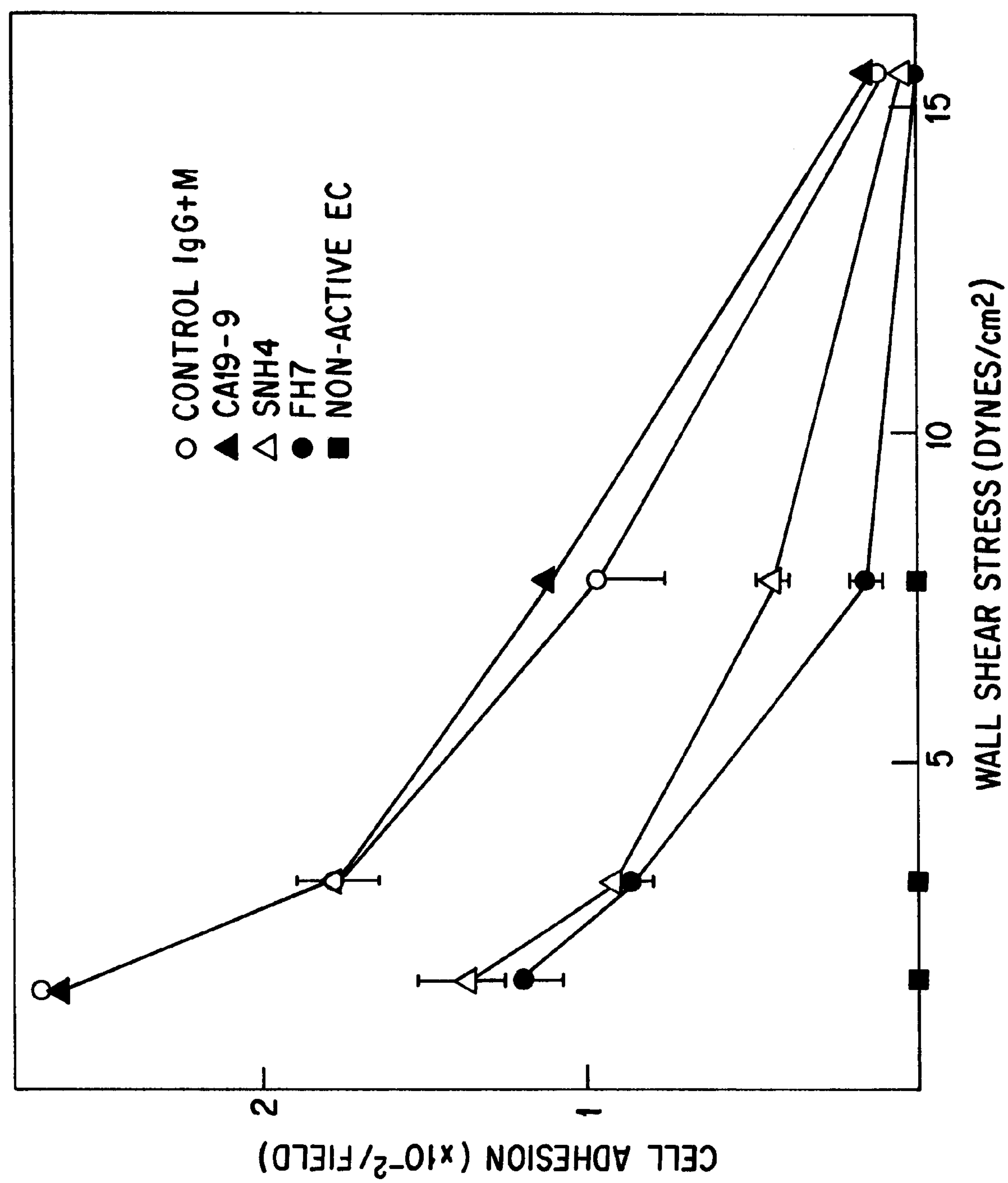
CONTROL IgG+M
CA19-9
SNH4
FH7
NON-ACTIVE EC
CELL ADHESION (x10^-2/FIELD)
2
1
WALL SHEAR STRESS (DYNES/cm²)
5
10
15

FIG.12